(19) Europäisches Patentamt
European Patent Office
Office européen des brevets

(11) EP 0 552 558 B1

(12) **EUROPEAN PATENT SPECIFICATION**

(45) Date of publication and mention of the grant of the patent:
**22.05.1996 Bulletin 1996/21**

(51) Int Cl.[6]: **C07D 249/10**, A01N 43/653

(21) Application number: **92311623.0**

(22) Date of filing: **18.12.1992**

(54) **1,5-Diphenyl-1H-1,2,4-triazole-3-carboxamide derivatives, process for preparation of the same, antifungal composition comprising the same**

1,5-Diphenyl-1,2,4-1H-Triazol-3-Carboxamidderivate, Verfahren zu ihrer Herstellung und diese enthaltende fungizide Zusammenstellungen

Dérivés de 1,5-diphényl-1H-1,2,4-triazole-3-carboxamide, procédé pour leur préparation et compositions fongicides les contenant

(84) Designated Contracting States:
**DE FR GB**

(30) Priority: **25.12.1991 JP 357007/91**

(43) Date of publication of application:
**28.07.1993 Bulletin 1993/30**

(73) Proprietor: **KUREHA CHEMICAL INDUSTRY CO., LTD.**
**Chuo-ku Tokyo 103 (JP)**

(72) Inventors:
- **Watanabe, Takeo**
  **Iwaki-shi, Fukushima (JP)**
- **Saishoji, Toshihide**
  **Iwaki-shi, Fukushima (JP)**
- **Shida, Takafumi**
  **Iwaki-shi, Fukushima (JP)**

(74) Representative: **Geering, Keith Edwin**
**REDDIE & GROSE**
**16 Theobalds Road**
**London WC1X 8PL (GB)**

(56) References cited:
**EP-A- 0 174 562** **EP-A- 0 282 303**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 552 558 B1

Printed by Jouve, 75001 PARIS (FR)

**Description**

BACKGROUND OF THE INVENTION

Field of the Invention

The present invention relates to a derivative of 1,5-diphenyl-1H-1,2,4-triazole-3-carboxamide, to a process for manufacturing the same, and to an antifungal composition, especially an antimicrobial composition used for agriculture and horticulture, comprising the same.

Description of Background Art

Prior art describing derivatives of 1,5-diphenyl-1H-1,2,4-triazole-3-carboxamide includes EP-A-282393, EP-A-282669, EP-A-220956.

All derivatives of 1,5-diphenyl-1H-1,2,4-triazole-3-carboxamide described in this prior art are herbicidal compounds, in which a substituted or unsubstituted alkoxymethyl group is bonded to the 3 position of 1-phenyl group of the triazole ring.

No derivatives of 1,5-diphenyl-1H-1,2,4-triazole-3-carboxamide represented by the following general formula (I) have been known in the art, nor have any studies been undertaken heretofore about their usefulness

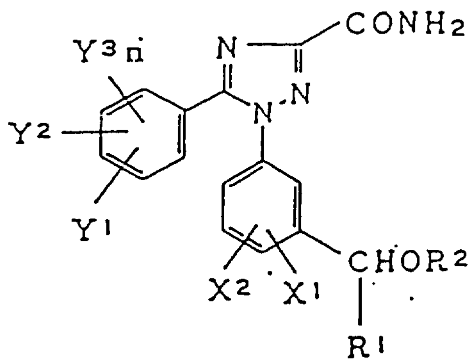

(I)

wherein $R^1$ is a $C_1$-$C_6$ alkyl group, $C_3$-$C_6$ cycloalkyl group, $C_1$-$C_5$ fluoroalkyl group, ($C_1$-$C_4$ alkoxy)methyl group, or phenyl group, $R_2$ is a $C_1$-$C_8$ alkyl group, ($C_3$-$C_6$ cycloalkyl)methyl group, $C_2$-$C_5$ fluoroalkyl group, ($C_1$-$C_4$ alkoxy)($C_1$-$C_4$ alkyl) group, phenyl group, phenylmethyl group, or phenylmethyl group substituted by a $C_1$-$C_4$ alkyl group or a halogen atom, $x^1$ is a hydrogen atom, a $C_1$-$C_4$ alkyl group, a $C_1$-$C_4$ alkoxy group, or a halogen atom, $X^2$ is a hydrogen atom, a $C_1$-$C_4$ alkyl group, or a halogen atom, $Y^1$ is a hydrogen atom, a halogen atom, a $C_1$-$C_4$ alkyl group, a $C_1$-$C_4$ alkoxy group, a $C_1$-$C_4$ fluoroalkoxy group, a HO group, a HOOC group or a ($C_1$-$C_4$ alkoxy)carbonyl group, $Y^2$ is a hydrogen atom, a $C_1$-$C_4$ alkyl group, or a halogen atom, $Y^3$ is a hydrogen atom or a halogen atom, and n is 1 or 2.

EP-A-174562 discloses 1-phenyl-1,2,4-triazole-3-carboxylic acid derivatives which are plant growth regulators or fungicides or protect against herbicidal damage, but in these the substituent at the 5-position is not substituted or unsubstituted phenyl.

The present inventors have found that, while derivatives of 1,5-diphenyl-1H-1,2,4-triazole-3-carboxamide, in which a substituted or unsubstituted alkoxy methyl group is bonded to the 3 position of 1-phenyl group of the triazole ring, which are described in EP-A-282303, EP-A-282669 and EP-A-220956,

are useful as herbicidal agents,the novel compounds of the general formula (I), in which a hydrogen atom of the methylene group is further substituted, are useful as antimicrobial agents.

The present invention provides derivatives of 1,5-diphenyl-1H-1,2,4-triazole-3-carboxamide represented by the general formula (I),

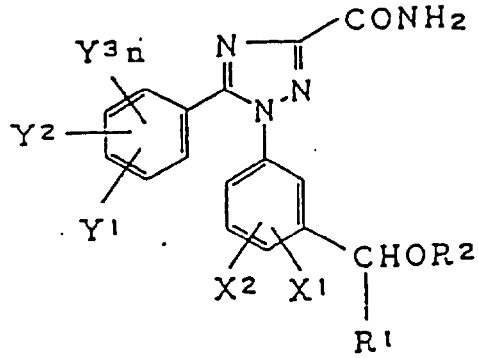

(I)

wherein $R^1$ is a $C_1$-$C_6$ alkyl group, $C_3$-$C_6$ cycloalkyl group, $C_1$-$C_5$ fluoroalkyl group, ($C_1$-$C_4$ alkoxy)methyl group, or phenyl group, $R^2$ is a $C_1$-$C_8$ alkyl group, ($C_3$-$C_6$ cycloalkyl)methyl group, $C_2$-$C_5$ fluoroalkyl group, ($C_1$-$C_4$ alkoxy)($C_1$-$C_4$ alkyl) group, phenyl group, phenylmethyl group, or phenylmethyl group substituted by a $C_1$-$C_4$ alkyl group or a halogen atom, $X^1$ is a hydrogen atom, a $C_1$-$C_4$ alkyl group, a $C_1$-$C_4$ alkoxy group, or a halogen atom, $X^2$ is a hydrogen atom, a $C_1$-$C_4$ alkoxy group, or a halogen atom, $Y^1$ is a hydrogen atom, a halogen atom, a $C_1$-$C_4$ alkyl group, a $C_1$-$C_4$ alkoxy group, a $C_1$-$C_4$ fluoroalkoxy group, a HO group, a HOOC group, or a ($C_1$-$C_4$ alkoxy)carbonyl group, $Y^2$ is a hydrogen atom, a $C_1$-$C_4$ alkyl group, or a halogen atom, $Y^3$ is a hydrogen atom or a halogen atom, and n is 1 or 2.

It also provides a process for manufacturing derivatives of 1,5-diphenyl-1H-1,2,4-triazole-3-carboxamide according to the following reaction formula (A)

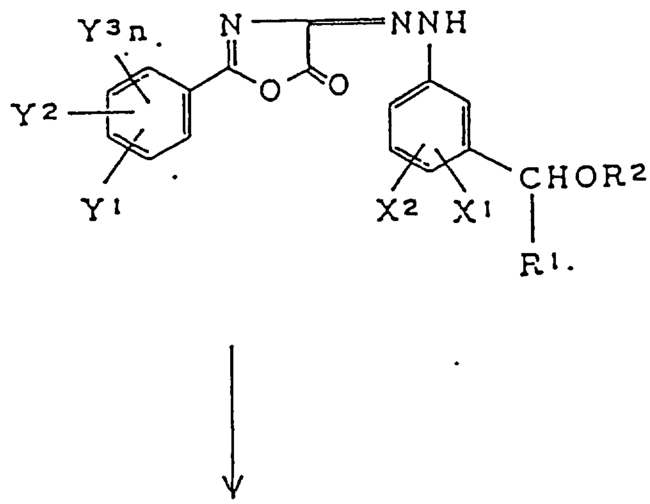

(VI)

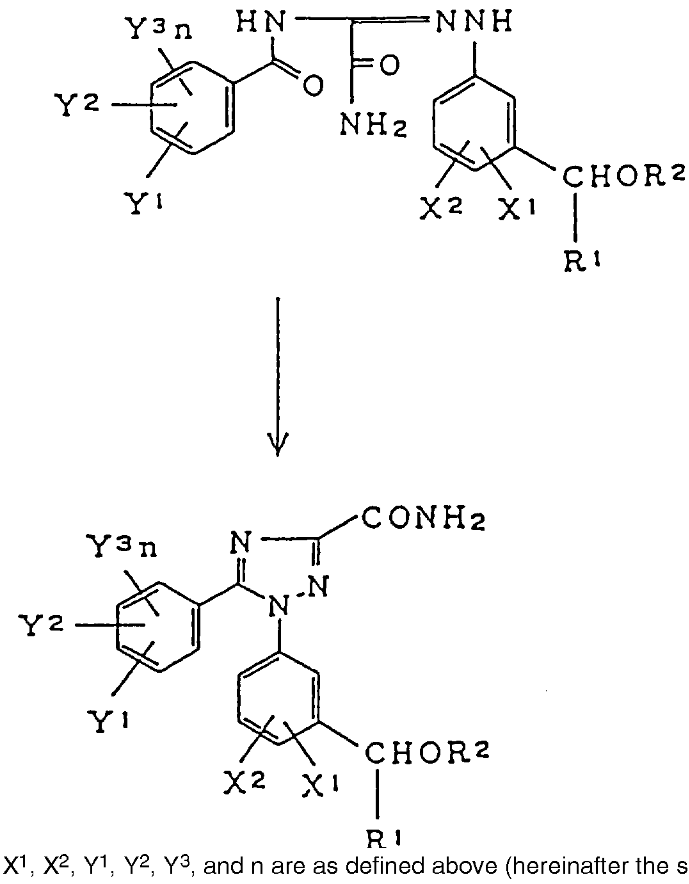

(VII)

(I)

wherein $R^1$, $R^2$, $X^1$, $X^2$, $Y^1$, $Y^2$, $Y^3$, and n are as defined above (hereinafter the same).

It further provides a process for manufacturing derivatives of 1,5-diphenyl-1H-1,2,4-triazole-3-carboxamide according to the following reaction formula (B)

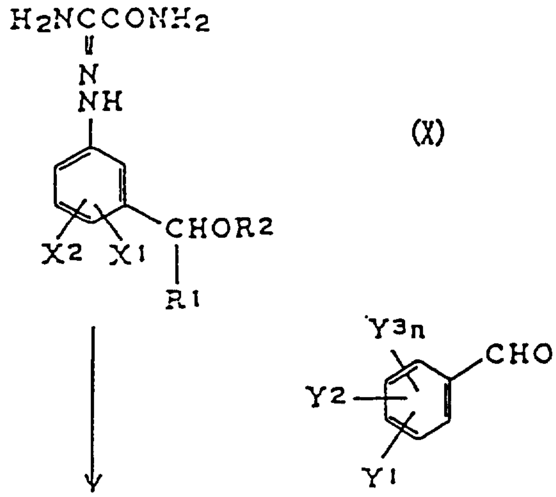

(X)

(XI)

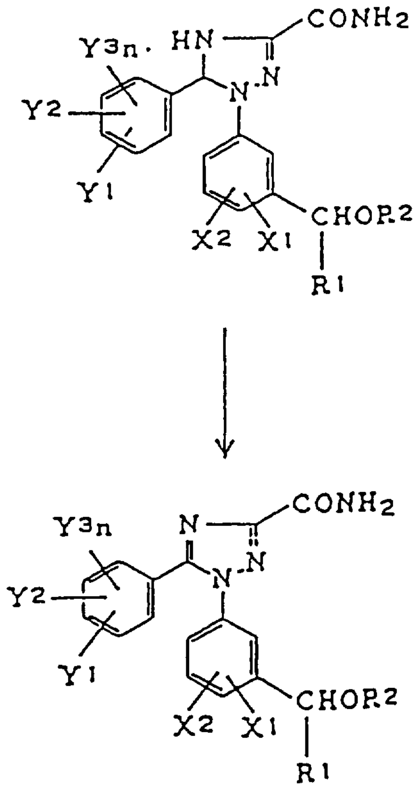

(XII)

(I)

It also provides an antifungal composition comprising a derivative of 1,5-diphenyl-1H-1,2,4-triazole-3-carboxamide of the general formula (I) as an effective ingredient.

In the above formulae it is preferred that $R^1$ is a $C_1$-$C_3$ alkyl group, $R^2$ is a $C_1$-$C_5$ alkyl group, $X^1$ is a hydrogen atom or a $C_1$-$C_2$ alkyl group, $X^2$ is a hydrogen atom, $Y^1$ is a hydrogen atom or a fluorine atom, $Y^2$ is a hydrogen atom or a fluorine atom, $Y^3$ is a hydrogen atom, and n is 1 or 2.

Given as specific examples of derivatives of 1,5-diphenyl-1H-1,2,4-triazole-3-carboxamide are compounds in the following Table 1. Physicochemical characteristics of these compounds are shown in Table 2.

TABLE 1

| Compound No. | $R^1$<br>$R^2$ | $X^1$<br>$X^2$ | $Y^1$<br>$Y^2$<br>$Y^3$ n |
|---|---|---|---|
| 1 | cyclopropyl<br>$n-C_4H_9$ | H<br>H | H<br>H<br>H |
| 2 | cyclopentyl<br>$n-C_4H_9$ | H<br>H | H<br>H<br>H |
| 3 | $t-C_4H_9$<br>$n-C_4H_9$ | H<br>H | 2-F<br>H<br>H |
| 4 | $t-C_4H_9$<br>$n-C_4H_9$ | H<br>H | H<br>H<br>H |
| 5 | $t-C_4H_9$<br>$PhCH_2$ | H<br>H | H<br>H<br>H |
| 6 | $n-C_3F_7$<br>$n-C_4H_9$ | $6-CH_3$<br>H | H<br>H<br>H |
| 7 | $C_2F_5$<br>$n-C_4H_9$ | H<br>H | H<br>H<br>H |
| 8 | $CF_3$<br>$n-C_4H_9$ | H<br>H | H<br>H<br>H |
| 9 | $n-C_4H_9$<br>$n-C_4H_9$ | H<br>H | H<br>H<br>H |
| 10 | $n-C_3H_7$<br>$n-C_4H_9$ | H<br>H | H<br>H<br>H |
| 11 | $C_2H_5$<br>$n-C_4H_9$ | $6-CH_3$<br>H | 2-F<br>3-F<br>H |

TABLE 1 (Continued)

| Compound No. | $R^1$ / $R^2$ | $X^1$ / $X^2$ | $Y^1$ / $Y^2$ / $Y^3$ n |
|---|---|---|---|
| 12 | $C_2H_5$ / $n\text{-}C_4H_9$ | 6-$CH_3$ / H | 2-F / H / H |
| 13 | $C_2H_5$ / $n\text{-}C_4H_9$ | 6-$CH_3$ / H | 2-F / 5-F / H |
| 14 | $C_2H_5$ / $n\text{-}C_4H_9$ | H / H | H / H / H |
| 15 | $C_2H_5$ / $n\text{-}C_4H_9$ | 6-$CH_3$ / H | H / H / H |
| 16 | $C_2H_5$ / $n\text{-}C_3H_7$ | H / H | H / H / H |
| 17 | $C_2H_5$ / $i\text{-}C_4H_9$ | H / H | H / H / H |
| 18 | $i\text{-}C_4H_9$ / $n\text{-}C_4H_9$ | H / H | H / H / H |
| 19 | $CH_3OCH_2$ / $n\text{-}C_4H_9$ | 6-$CH_3$ / H | H / H / H |
| 20 | $CH_3$ / $(n\text{-}C_3F_7)CH_2$ | H / H | H / H / H |
| 21 | $CH_3$ / $n\text{-}C_6H_{13}$ | H / H | H / H / H |
| 22 | $CH_3$ / $n\text{-}C_5H_{11}$ | H / H | H / H / H |
| 23 | $CH_3$ / $n\text{-}C_4H_9$ | H / H | 2-F / H / H |

TABLE 1 (Continued)

| Compound No. | $R^1$ / $R^2$ | $X^1$ / $X^2$ | $Y^1$ / $Y^2$ / $Y^3$ n |
|---|---|---|---|
| 24 | $CH_3$<br>n-$C_4H_9$ | 6-Cl<br>H | 3-F<br>5-F<br>H |
| 25 | $CH_3$<br>n-$C_4H_9$ | 6-Cl<br>H | 2-F<br>H<br>H |
| 26 | $CH_3$<br>n-$C_4H_9$ | 6-Cl<br>H | 2-F<br>3-F<br>5,6-F2 |
| 27 | $CH_3$<br>n-$C_4H_9$ | 6-Cl<br>H | 2-F<br>3-F<br>H |
| 28 | $CH_3$<br>n-$C_4H_9$ | 6-Cl<br>H | 2-F<br>5-F<br>H |
| 29 | $CH_3$<br>n-$C_4H_9$ | 6-Cl<br>H | H<br>H<br>H |
| 30 | $CH_3$<br>n-$C_4H_9$ | 4-$C_2H_5O$<br>H | H<br>H<br>H |
| 31 | $CH_3$<br>n-$C_4H_9$ | 4-$CH_3$<br>H | H<br>H<br>H |
| 32 | $CH_3$<br>n-$C_4H_9$ | 4-Cl<br>H | H<br>H<br>H |
| 33 | $CH_3$<br>n-$C_4H_9$ | 6-$CH_3O$<br>H | H<br>H<br>H |
| 34 | $CH_3$<br>n-$C_4H_9$ | 6-Cl<br>H | H<br>H<br>H |
| 35 | $CH_3$<br>n-$C_4H_9$ | 6-$CH_3$<br>H | H<br>H<br>H |

TABLE 1 (Continued)

| Compound No. | $R^1$ / $R^2$ | $X^1$ / $X^2$ | $Y^1$ / $Y^2$ / $Y^3$ n |
|---|---|---|---|
| 36 | $CH_3$<br>$n-C_4H_9$ | H<br>H | H<br>H<br>H |
| 37 | $CH_3$<br>$n-C_3H_7$ | H<br>H | H<br>H<br>H |
| 38 | $CH_3$<br>$i-C_5H_{11}$ | H<br>H | H<br>H<br>H |
| 39 | $CH_3$<br>$CH_3$ | 4-Cl<br>H | H<br>H<br>H |
| 40 | $CH_3$<br>$CH_3$ | H<br>H | H<br>H<br>H |
| 41 | $s-C_4H_9$<br>$n-C_4H_9$ | H<br>H | H<br>H<br>H |
| 42 | $i-C_3H_7$<br>$(cyclohexyl)CH_2$ | H<br>H | H<br>H<br>H |
| 43 | $i-C_3H_7$<br>$n-C_5H_{11}$ | H<br>H | 2-F<br>H<br>H |
| 44 | $i-C_3H_7$<br>$n-C_5H_{11}$ | H<br>H | H<br>H<br>H |
| 45 | $i-C_3H_7$<br>$n-C_4H_9$ | H<br>H | $2-CH_3$<br>H<br>H |
| 46 | $i-C_3H_7$<br>$n-C_4H_9$ | H<br>H | $3-CH_3$<br>H<br>H |
| 47 | $i-C_3H_7$<br>$n-C_4H_9$ | H<br>H | $4-CH_3$<br>H<br>H |

TABLE 1 (Continued)

| Compound No. | $R^1$ / $R^2$ | $X^1$ / $X^2$ | $Y^1$ / $Y^2$ / $Y^3$ n |
|---|---|---|---|
| 48 | i-$C_3H_7$<br>n-$C_4H_9$ | 6-$CH_3$<br>H | 2-$COOCH_3$<br>H<br>H |
| 49 | i-$C_3H_7$<br>n-$C_4H_9$ | 6-$CH_3$<br>H | 2-COOH<br>H<br>H |
| 50 | i-$C_3H_7$<br>n-$C_4H_9$ | H<br>H | 2-Cl<br>H<br>H |
| 51 | i-$C_3H_7$<br>n-$C_4H_9$ | H<br>H | 2-Cl<br>6-F<br>H |
| 52 | i-$C_3H_7$<br>n-$C_4H_9$ | H<br>H | 4-Cl<br>H<br>H |
| 53 | i-$C_3H_7$<br>n-$C_4H_9$ | 6-$CH_3$<br>H | 2-F<br>H<br>H |
| 54 | i-$C_3H_7$<br>n-$C_4H_9$ | 6-$CH_3$<br>H | 2-F<br>3-F<br>H |
| 55 | i-$C_3H_7$<br>n-$C_4H_9$ | H<br>H | 2-F<br>4-F<br>H |
| 56 | i-$C_3H_7$<br>n-$C_4H_9$ | H<br>H | 2-F<br>6-F<br>H |
| 57 | i-$C_3H_7$<br>n-$C_4H_9$ | H<br>H | 2-F<br>3-F<br>H |
| 58 | i-$C_3H_7$<br>n-$C_4H_9$ | H<br>H | 2-F<br>H<br>H |
| 59 | i-$C_3H_7$<br>n-$C_4H_9$ | 6-$CH_3$<br>H | 2-F<br>5-F<br>H |

TABLE 1 (Continued)

| Compound No. | $R^1$<br>$R^2$ | $X^1$<br>$X^2$ | $Y^1$<br>$Y^2$<br>$Y^3$ n |
|---|---|---|---|
| 60 | i-$C_3H_7$<br>n-$C_4H_9$ | H<br>H | 2-F<br>5-F<br>H |
| 61 | i-$C_3H_7$<br>n-$C_4H_9$ | H<br>H | 3-F<br>H<br>H |
| 62 | i-$C_3H_7$<br>n-$C_4H_9$ | H<br>H | 3-F<br>4-F<br>H |
| 63 | i-$C_3H_7$<br>n-$C_4H_9$ | 6-$CH_3$<br>H | 3-F<br>5-F<br>H |
| 64 | i-$C_3H_7$<br>n-$C_4H_9$ | H<br>H | 4-F<br>H<br>H |
| 65 | i-$C_3H_7$<br>n-$C_4H_9$ | H<br>H | H<br>H<br>H |
| 66 | i-$C_3H_7$<br>n-$C_4H_9$ | 6-$C_2H_5$<br>H | H<br>H<br>H |
| 67 | i-$C_3H_7$<br>n-$C_4H_9$ | 6-$CH_3$<br>H | H<br>H<br>H |
| 68 | i-$C_3H_7$<br>n-$C_4H_9$ | 6-i-$C_3H_7$<br>H | H<br>H<br>H |
| 69 | i-$C_3H_7$<br>n-$C_4H_9$ | 6-Cl<br>H | H<br>H<br>H |
| 70 | i-$C_3H_7$<br>n-$C_4H_9$ | H<br>H | 2-$CF_3O$<br>H<br>H |
| 71 | i-$C_3H_7$<br>n-$C_4H_9$ | H<br>H | 2-$C_2H_5O$<br>H<br>H |

TABLE 1 (Continued)

| Compound No. | $R^1$<br>$R^2$ | $X^1$<br>$X^2$ | $Y^1$<br>$Y^2$<br>$Y^3$ n |
|---|---|---|---|
| 72 | $i-C_3H_7$<br>$n-C_4H_9$ | H<br>H | $2-CH_3O$<br>H<br>H |
| 73 | $i-C_3H_7$<br>$n-C_4H_9$ | H<br>H | $3-CH_3O$<br>H<br>H |
| 74 | $i-C_3H_7$<br>$n-C_4H_9$ | H<br>H | $4-CH_3O$<br>H<br>H |
| 75 | $i-C_3H_7$<br>$n-C_3H_7$ | H<br>H | H<br>H<br>H |
| 76 | $i-C_3H_7$<br>$i-C_5H_{11}$ | H<br>H | H<br>H<br>H |
| 77 | $i-C_3H_7$<br>$C_2H_5OC_2H_4$ | H<br>H | H<br>H<br>H |
| 78 | $i-C_3H_7$<br>$C_2H_5$ | H<br>H | H<br>H<br>H |
| 79 | $i-C_3H_7$<br>$PhCH_2$ | H<br>H | 2-F<br>H<br>H |
| 80 | $i-C_3H_7$<br>$PhCH_2$ | H<br>H | 2-F<br>3-F<br>H |
| 81 | $i-C_3H_7$<br>$PhCH_2$ | H<br>H | 2-F<br>5-F<br>H |
| 82 | $i-C_3H_7$<br>$PhCH_2$ | H<br>H | H<br>H<br>H |
| 83 | $i-C_3H_7$<br>$PhCH_2$ | H<br>H | 2-HO<br>H<br>H |

TABLE 1 (Continued)

| Compound No. | $R^1$<br>$R^2$ | $X^1$<br>$X^2$ | $Y^1$<br>$Y^2$<br>$Y^3$ n |
|---|---|---|---|
| 84 | $i-C_3H_7$<br>$(2-CH_3-Ph)CH_2$ | H<br>H | H<br>H<br>H |
| 85 | $i-C_3H_7$<br>$(4-Cl-Ph)CH_2$ | H<br>H | H<br>H<br>H |
| 86 | $i-C_3H_7$<br>Ph | H<br>H | H<br>H<br>H |
| 87 | Ph<br>$n-C_4H_9$ | H<br>H | H<br>H<br>H |

TABLE 2

| Compound No. | Physicochemical Characteristics |
|---|---|
| 1 | Appearance : Light yellow liquid<br>IR ($cm^{-1}$) : 3450, 3290, 1690 |
| 2 | Appearance : Colorless crystal<br>m.p. (°C) : 101-103<br>IR ($cm^{-1}$) : 3420, 3300, 1680 |
| 3 | Appearance : Light yellow crystal<br>m.p. (°C) : 75-77<br>IR ($cm^{-1}$) : 3410, 3310, 1670 |
| 4 | Appearance : Light yellow crystal<br>m.p. (°C) : 96-98<br>IR ($cm^{-1}$) : 3450, 3300, 1680 |
| 5 | Appearance : Colorless crystal<br>m.p. (°C) : 146-148<br>IR ($cm^{-1}$) : 3450, 3290, 1680 |
| 6 | Appearance : Light brown liquid<br>IR ($cm^{-1}$) : 3440, 3300, 1675 |

TABLE 2 (Continued)

| Compound No. | Physicochemical Characteristics |
|---|---|
| 7 | Appearance : Colorless crystal<br>m.p. (°C) : 132-134<br>IR ($cm^{-1}$) : 3450, 3370, 1665 |
| 8 | Appearance : Yellow crystal<br>m.p. (°C) : 72-74<br>IR ($cm^{-1}$) : 3440, 3320, 1680 |
| 9 | Appearance : Colorless crystal<br>m.p. (°C) : 87-89<br>IR ($cm^{-1}$) : 3400, 3290, 1670 |
| 10 | Appearance : Light brown crystal<br>m.p. (°C) : 80-82<br>IR ($cm^{-1}$) : 3450, 3280, 1680 |
| 11 | Appearance : Light brown liquid<br>IR ($cm^{-1}$) : 3450, 3170, 1680 |
| 12 | Appearance : Colorless crystal<br>m.p. (°C) : 103-105<br>IR ($cm^{-1}$) : 3440, 3160, 1680 |
| 13 | Appearance : Light brown liquid<br>IR ($cm^{-1}$) : 3450, 3160, 1680 |
| 14 | Appearance : Light brown crystal<br>m.p. (°C) : 68-70<br>IR ($cm^{-1}$) : 3450, 3300, 1690 |
| 15 | Appearance : Light brown crystal<br>m.p. (°C) : 140-141<br>IR ($cm^{-1}$) : 3450, 3130, 1690 |
| 16 | Appearance : Light brown liquid<br>IR ($cm^{-1}$) : 3450, 3260, 1680 |
| 17 | Appearance : Light yellow liquid<br>IR ($cm^{-1}$) : 3450, 3290, 1690 |
| 18 | Appearance : Light reddish brown crystal<br>m.p. (°C) : 108-110<br>IR ($cm^{-1}$) : 3440, 3375, 1670 |

TABLE 2 (Continued)

| Compound No. | Physicochemical Characteristics |
| --- | --- |
| 19 | Appearance : Light brown liquid<br>IR ($cm^{-1}$) : 3450, 3290, 1675 |
| 20 | Appearance : Light brown liquid<br>IR ($cm^{-1}$) : 3440, 3360, 1670 |
| 21 | Appearance : Light brown liquid<br>IR ($cm^{-1}$) : 3450, 3310, 1690 |
| 22 | Appearance : Colorless crystal<br>m.p. (°C) : 50-52<br>IR ($cm^{-1}$) : 3450, 3290, 1690 |
| 23 | Appearance : Light yellow liquid<br>IR ($cm^{-1}$) : 3450, 3300, 1690 |
| 24 | Appearance : Light yellow crystal<br>m.p. (°C) : 108-110<br>IR ($cm^{-1}$) : 3470, 3300, 1670 |
| 25 | Appearance : Light brown liquid<br>IR ($cm^{-1}$) : 3450, 3160, 1680 |
| 26 | Appearance : Colorless crystal<br>m.p. (°C) : 130-132<br>IR ($cm^{-1}$) : 3450, 3175, 1685 |
| 27 | Appearance : Light brown liquid<br>IR ($cm^{-1}$) : 3450, 3160, 1680 |
| 28 | Appearance : Light brown liquid<br>IR ($cm^{-1}$) : 3450, 3170, 1685 |
| 29 | Appearance : Light brown crystal<br>m.p. (°C) : 66-68<br>IR ($cm^{-1}$) : 3430, 3300, 1670 |
| 30 | Appearance : Light brown crystal<br>m.p. (°C) : 180-182<br>IR ($cm^{-1}$) : 3420, 3300, 1690, 1665 |

TABLE 2 (Continued)

| Compound No. | Physicochemical Characteristics |
|---|---|
| 31 | Appearance : Colorless crystal<br>m.p. (°C) : 129-131<br>IR ($cm^{-1}$) : 3460, 3230, 1700, 1670 |
| 32 | Appearance : Light brown crystal<br>m.p. (°C) : 129-131<br>IR ($cm^{-1}$) : 3450, 3300, 1675 |
| 33 | Appearance : Light brown crystal<br>m.p. (°C) : 168-170<br>IR ($cm^{-1}$) : 3480, 3300, 1710, 1670 |
| 34 | Appearance : Colorless crystal<br>m.p. (°C) : 130-132<br>IR ($cm^{-1}$) : 3450, 3130, 1690 |
| 35 | Appearance : Light brown crystal<br>m.p. (°C) : 155-157<br>IR ($cm^{-1}$) : 3450, 3125, 1690 |
| 36 | Appearance : Colorless crystal<br>m.p. (°C) : 92-94<br>IR ($cm^{-1}$) : 3470, 3240, 1715, 1675 |
| 37 | Appearance : Colorless crystal<br>m.p. (°C) : 86-88<br>IR ($cm^{-1}$) : 3450, 3290, 1675 |
| 38 | Appearance : Colorless crystal<br>m.p. (°C) : 96-98<br>IR ($cm^{-1}$) : 3470, 3270, 1710, 1670 |
| 39 | Appearance : Light brown crystal<br>m.p. (°C) : 117-119<br>IR ($cm^{-1}$) : 3380, 3230, 1680 |
| 40 | Appearance : Colorless crystal<br>m.p. (°C) : 174-176<br>IR ($cm^{-1}$) : 3450, 3240, 1710, 1675 |
| 41 | Appearance : Colorless crystal<br>m.p. (°C) : 74-76<br>IR ($cm^{-1}$) : 3450, 3300, 1675 |
| 42 | Appearance : Colorless crystal<br>m.p. (°C) : 147-149<br>IR ($cm^{-1}$) : 3475, 3310, 1715, 1670 |

TABLE 2 (Continued)

| Compound No. | Physicochemical Characteristics |
|---|---|
| 43 | Appearance : Light yellow liquid<br>IR ($cm^{-1}$) : 3460, 3300, 1675 |
| 44 | Appearance : Light yellow crystal<br>m.p. (°C) : 81-83<br>IR ($cm^{-1}$) : 3400, 3300, 1675 |
| 45 | Appearance : Light brown liquid<br>IR ($cm^{-1}$) : 3460, 3275, 1680 |
| 46 | Appearance : Light brown liquid<br>IR ($cm^{-1}$) : 3460, 3300, 1680 |
| 47 | Appearance : Light brown liquid<br>IR ($cm^{-1}$) : 3400, 3300, 1680 |
| 48 | Appearance : Colorless liquid<br>IR ($cm^{-1}$) : 3450, 3300, 1730, 1670 |
| 49 | Appearance : Light brown crystal<br>m.p. (°C) : 132-134<br>IR ($cm^{-1}$) : 3450, 3290, 1700, 1675 |
| 50 | Appearance : Light brown crystal<br>m.p. (°C) : 73-75<br>IR ($cm^{-1}$) : 3400, 3310, 1680, 1665 |
| 51 | Appearance : Light brown liquid<br>IR ($cm^{-1}$) : 3450, 3170, 1680 |
| 52 | Appearance : Brown liquid<br>IR ($cm^{-1}$) : 3450, 3270, 1690 |
| 53 | Appearance : Light brown liquid<br>IR ($cm^{-1}$) : 3450, 3200, 1680 |
| 54 | Appearance : Colorless crystal<br>m.p. (°C) : 97-98<br>IR ($cm^{-1}$) : 3360, 3250, 1670 |

TABLE 2 (Continued)

| Compound No. | Physicochemical Characteristics |
|---|---|
| 55 | Appearance : Light brown crystal<br>m.p. (°C) : 120-122<br>IR ($cm^{-1}$) : 3450, 3300, 1675 |
| 56 | Appearance : Light brown liquid<br>IR ($cm^{-1}$) : 3450, 3300, 1690 |
| 57 | Appearance : Light brown liquid<br>IR ($cm^{-1}$) : 3450, 3280, 1680 |
| 58 | Appearance : Colorless crystal<br>m.p. (°C) : 79-81<br>IR ($cm^{-1}$) : 3450, 3300, 1700 |
| 59 | Appearance : Brown liquid<br>IR ($cm^{-1}$) : 3450, 3170, 1685 |
| 60 | Appearance : Colorless crystal<br>m.p. (°C) : 139-141<br>IR ($cm^{-1}$) : 3470, 3210, 1705, 1670 |
| 61 | Appearance : Colorless crystal<br>m.p. (°C) : 106-108<br>IR ($cm^{-1}$) : 3475, 3230, 1710, 1665 |
| 62 | Appearance : Light brown liquid<br>IR ($cm^{-1}$) : 3450, 3170, 1670 |
| 63 | Appearance : Brown liquid<br>IR ($cm^{-1}$) : 3450, 3170, 1685 |
| 64 | Appearance : Light yellow crystal<br>m.p. (°C) : 53-55<br>IR ($cm^{-1}$) : 3440, 3225, 1670 |
| 65 | Appearance : Light brown crystal<br>m.p. (°C) : 87-89<br>IR ($cm^{-1}$) : 3430, 3290, 1680 |
| 66 | Appearance : Light yellow liquid<br>IR ($cm^{-1}$) : 3440, 3300, 1680 |

TABLE 2 (Continued)

| Compound No. | Physicochemical Characteristics |
|---|---|
| 67 | Appearance : Colorless crystal<br>m.p. (°C) : 134-135<br>IR ($cm^{-1}$) : 3400, 3230, 1670 |
| 68 | Appearance : Light yellow liquid<br>IR ($cm^{-1}$) : 3450, 3300, 1675 |
| 69 | Appearance : Light brown crystal<br>m.p. (°C) : 66-68<br>IR ($cm^{-1}$) : 3445, 3160, 1690 |
| 70 | Appearance : Light yellow crystal<br>m.p. (°C) : 106-108<br>IR ($cm^{-1}$) : 3450, 3295, 1680 |
| 71 | Appearance : Colorless crystal<br>m.p. (°C) : 113-115<br>IR ($cm^{-1}$) : 3350, 3170, 1695, 1665 |
| 72 | Appearance : Light brown crystal<br>m.p. (°C) : 94-96<br>IR ($cm^{-1}$) : 3375, 3300, 1680 |
| 73 | Appearance : Colorless crystal<br>m.p. (°C) : 117-119<br>IR ($cm^{-1}$) : 3440, 3180, 1680 |
| 74 | Appearance : Yellow liquid<br>IR ($cm^{-1}$) : 3450, 3290, 1685 |
| 75 | Appearance : Light brown liquid<br>IR ($cm^{-1}$) : 3450, 3300, 1670 |
| 76 | Appearance : Colorless crystal<br>m.p. (°C) : 102-104<br>IR ($cm^{-1}$) : 3400, 3290, 1670 |
| 77 | Appearance : Brown liquid<br>IR ($cm^{-1}$) : 3440, 3310, 1670 |
| 78 | Appearance : Light brown crystal<br>m.p. (°C) : 79-81<br>IR ($cm^{-1}$) : 3450, 3295, 1675 |

TABLE 2 (Continued)

| Compound No. | Physicochemical Characteristics |
|---|---|
| 79 | Appearance : Light yellow crystal<br>m.p. (°C) : 128-130<br>IR ($cm^{-1}$) : 3475, 3325, 1670 |
| 80 | Appearance : Light yellow liquid<br>IR ($cm^{-1}$) : 3450, 3250, 1680 |
| 81 | Appearance : Light yellow liquid<br>IR ($cm^{-1}$) : 3460, 3270, 1680 |
| 82 | Appearance : Light brown liquid<br>IR ($cm^{-1}$) : 3450, 3270, 1685 |
| 83 | Appearance : Gray crystal<br>m.p. (°C) : 178-180<br>IR ($cm^{-1}$) : 3430, 3140, 1650 |
| 84 | Appearance : Light brown crystal<br>m.p. (°C) : 114-116<br>IR ($cm^{-1}$) : 3470, 3300, 1670 |
| 85 | Appearance : Light brown liquid<br>IR ($cm^{-1}$) : 3450, 3270, 1680 |
| 86 | Appearance : Colorless crystal<br>m.p. (°C) : 114-116<br>IR ($cm^{-1}$) : 3450, 3300, 1670 |
| 87 | Appearance : Light brown liquid<br>IR ($cm^{-1}$) : 3450, 3275, 1690 |

For the preparation of derivatives of 1,5-diphenyl-1H-1,2,4-triazole-3-carboxamide of formula (I), a derivative of oxazoledione hydrazone of formula (VI) and ammonia are reacted in an organic solvent to produce a derivative of hippuric acid amide of formula (VII), followed by a cyclocondensation reaction by an acid catalyst in an organic solvent according to the above-described reaction formula (A) [hereinafter called Process (A)], or an oxamide derivative of formula (X) and benzaldehyde derivative of formula (XI) are reacted in an organic solvent to produce dihydrotriazolecarboxamide of formula (XII), followed by oxidation of the dihydrotriazolecarboxamide according to the above-described reaction formula (B) [hereinafter called Process (B)].

Processes for producing the oxazoledione hydrazone derivative of formula (VI) used in Process (A) are discussed in detail.

(Diazotization and diazo coupling reaction)

The oxazoledione hydrazone derivative of formula (VI) can be produced according to the following reaction formula

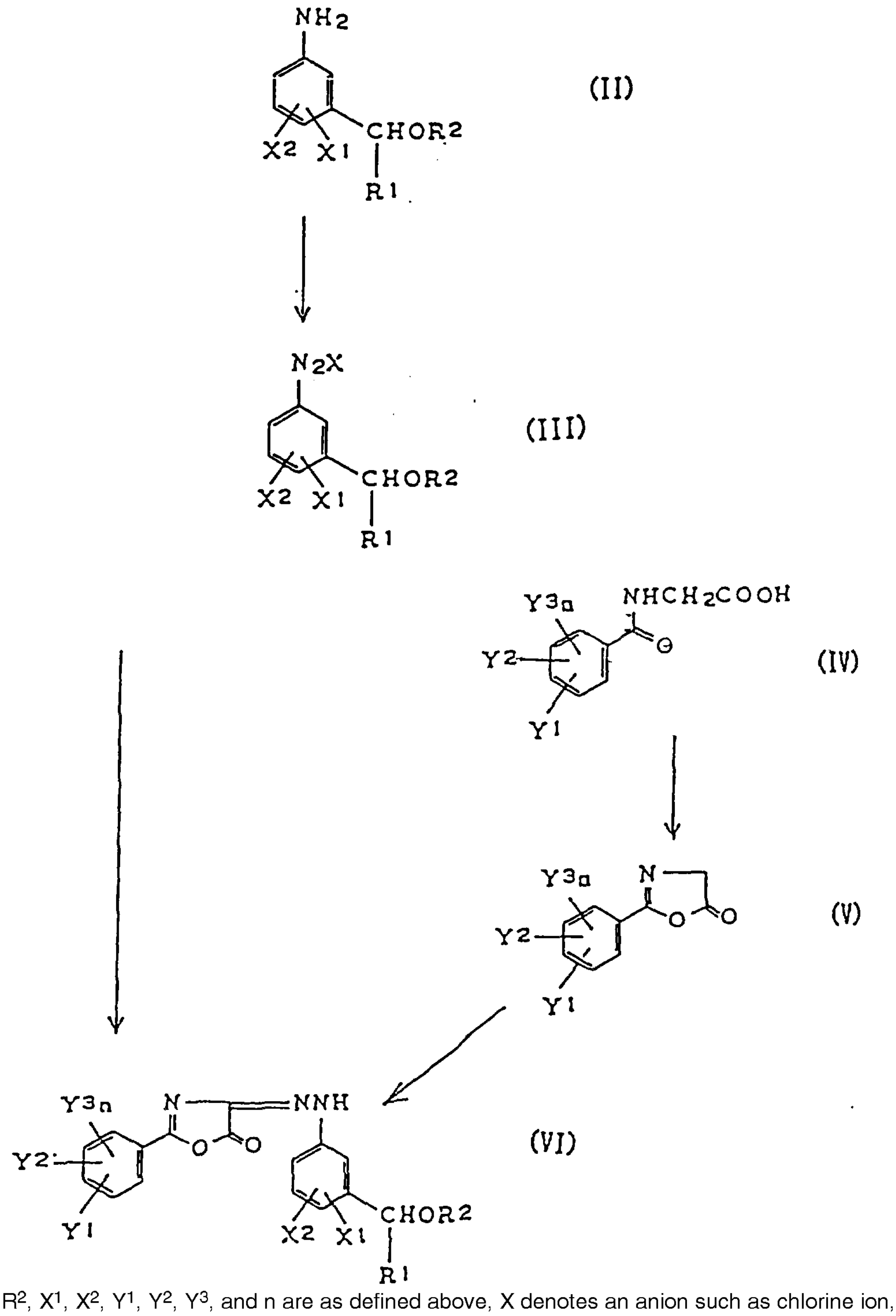

(II)

(III)

(IV)

(V)

(VI)

wherein $R^1$, $R^2$, $X^1$, $X^2$, $Y^1$, $Y^2$, $Y^3$, and n are as defined above, X denotes an anion such as chlorine ion, sulfate ion, or boron tetrafluoride ion.

According to the above reaction formula, an aniline derivative of formula (II) is converted to a diazonium salt derivative of formula (III) by adding dropwise an aqueous solution of nitrite such as sodium nitrite into a mixture of the aniline derivative of formula (II), an inorganic acid, e.g., hydrochloric acid, sulfuric acid, etc., and an organic acid, e.g., acetic acid, propionic acid, etc., while cooling and with stirring. The diazonium salt derivative of formula (III) thus obtained is reacted with a hippuric acid derivative of formula (IV), acetic anhydride, and a basic compound to produce a derivative of oxazoledione hydrazone of formula (VI). Given as basic compounds used here are alkali metal salts of weak acid, e.g., sodium acetate, etc., alkaline earth metal salts of weak acid, etc., e.g., calcium acetate, alkaline earth metal oxides, e.g., calcium oxide, magnesium oxide, etc., and tertiary amines, e.g., pyridine derivatives, triethylamine, tripropylamine, etc. Alternatively, the hippuric acid derivative of formula (IV) may be used for the diazo coupling reaction after converting it into the oxazolone derivative of formula (V) by heating together with acetic anhydride.

(Acylation reaction, nitration reaction, and reduction of carbonyl group)

Aniline derivative of formula (II) can be obtained via nitrobenzyl alcohol derivative of formula (XVII) which is produced by the following reaction.

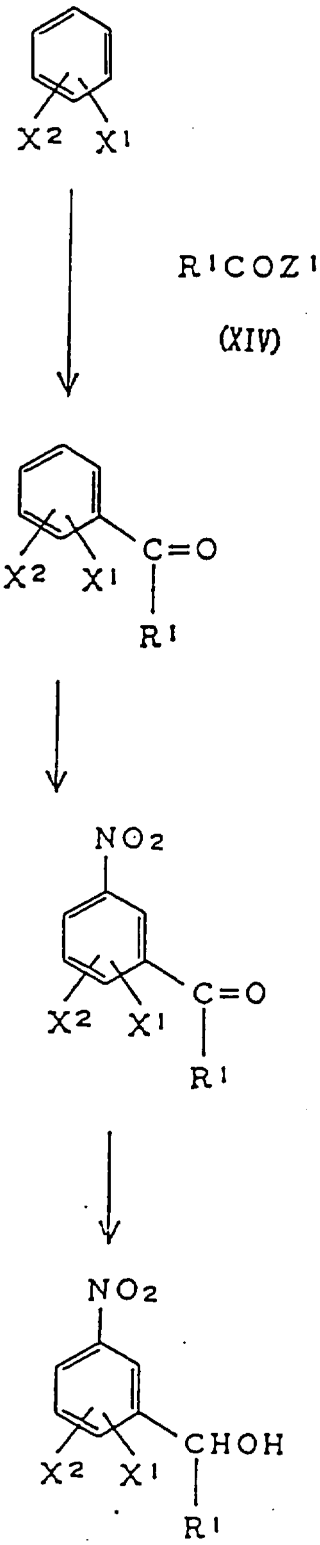


(XIII)

(XV)

(XVI)

(XVII)

wherein $R^1$, $X^1$, and $X^2$ are as defined above, and $Z^1$ represents a halogen atom or a group $R^1COO$, preferably Cl or Br.

According to the above reaction formula benzene derivative of formula (XIII) is acylated by acyl halide of formula (XIV) or carboxylic acid anhydride in the presence of Lewis acid, e.g., $AlCl_3$, $AlBr_3$, $ZnCl_2$, or $BF_3$, preferably $AlCl_3$, to produce ketone derivative of formula (XV). In this reaction, an excessive amount of acylation substrate or an organic

solvent, e.g., nitrobenzene, carbon disulfide, etc., can be used as a reaction solvent. The ketone derivative of formula (XV) is converted into nitrophenyl ketone derivative of formula (XVI) by nitration using nitric acid in a mixture of nitric acid and sulfuric acid or in acetic acid solvent. Nitrobenzyl alcohol derivative of formula (XVII) can be produced by reducing the carbonyl group of the nitrophenyl ketone derivative of formula (XVI) thus obtained. The use of sodium borohydride as a reducing agent and an alcohol, e.g., ethyl alcohol, is preferred for the reducing reaction.

(Etherification reaction and nitro group reducing reaction)

According to the reaction formula shown below, nitrobenzyl alcohol derivative of formula (XVII) is etherified with an alkylating agent represented by formula (XVIII), such as alkyl halide, alkyl sulfonate, or the like, to produce nitrobenzyl ether derivative of formula (XIX). An acid-binding agent used in this reaction is a basic compound such as, for example, hydroxide or carbonate of alkali metal, hydroxide, oxide or carbonate of alkaline earth metal, or the like, preferably potassium hydroxide, sodium hydride, potassium carbonate, and the like. The reaction is carried out preferably in the presence of a solvent, such as an aprotic polar solvent, e.g., dimethylformamide, dimethylacetamide, N-methylpyrrolidone, dimethylsulfoxide, tetrahydrofuran, sulfolane, acetonitrile, etc. The reaction temperature is usually -10 to 100°C, and the reaction time is 0.5 to 25 hours, and preferably 1 to 10 hours. The nitro group in the nitrobenzyl ether derivative of formula (XIX) thus obtained is reduced to produce aniline derivative of formula (II). Bechamp reduction agent, e.g., hydrochloric acid and metallic iron; tin (I) chloride; catalytic hydrogenation agent, e.g., hydrogen molecule catalyzed by platinum/activated charcoal, palladium/activated charcoal, etc.; hydrazine hydrate catalyzed by activated charcoal and iron (II) chloride; or the like can be used in the reduction. The solvent used differs depending on the reduction method employed. An organic acid, e.g., acetic acid, propionic acid, etc., can be used for the Bechamp reduction; an alcohol, e.g., ethyl alcohol, isopropyl alcohol, etc., an aromatic hydrocarbon, e.g., benzene, toluene, etc., or the like can be used for the catalytic reduction. The reaction temperature is usually -10 to 100°C, and the reaction time is 0.5 to 24 hours, and preferably 1 to 10 hours;

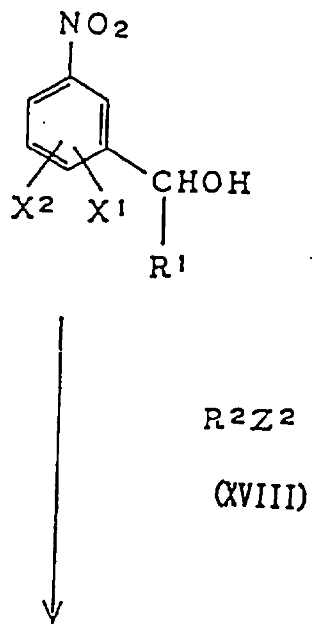


(XVII)

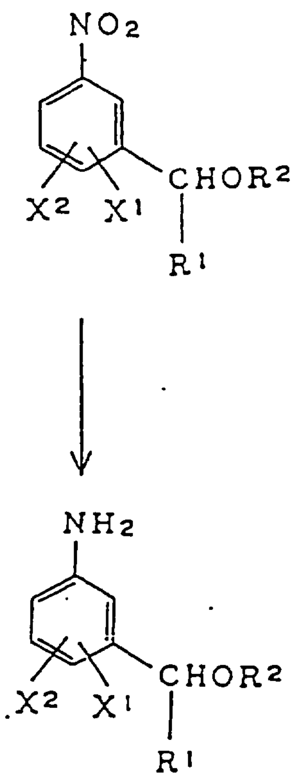

(XIX)

(II)

wherein $R^1$, $R^2$, $X^1$, and $X^2$ are as defined above, and $Z^2$ represents a halogen atom or a group $QSO_2O$, wherein Q is a $C_1$-$C_4$ alkyl group, substituted or unsubstituted phenyl group, preferably methyl group, phenyl group, or p-methylphenyl group.

Process (B) is now discussed in detail. (Diazotization and Japp-Klingemann reaction)

Oxamide derivative of formula (X) can be prepared as follows. According to the reaction formula shown below, an aniline derivative of formula (II) is converted to a diazonium salt derivative of formula (III) by adding dropwise an aqueous solution of nitrite such as sodium nitrite into a mixture of the aniline derivative of formula (II) and an inorganic acid, e.g., hydrochloric acid, sulfuric acid, etc., and a lower alcohol, e.g., methyl alcohol, etc., while cooling and with stirring. The diazonium salt derivative of formula (III) is then reacted with a 2-chloroacetoacetate derivative of formula (VIII) and a basic compound, preferably in a lower alcohol such as methanol or the like, to obtain oxalyl chloride derivative of formula (IX).

Given as basic compounds used here are alkali metal salts of weak acid, e.g., sodium acetate, etc., alkaline earth metal salts of weak acid, e.g., calcium acetate, alkaline earth metal oxides, e.g., calcium oxide, magnesium oxide, etc., tertiary amines, e.g., pyridine derivatives, triethylamine, tripropylamine, etc.

The oxalyl chloride derivative and ammonia are reacted in a lower alcohol such as methanol or the like at -20 to 50°C for 10 to 30 hours to obtain an oxamide derivative of formula (X)

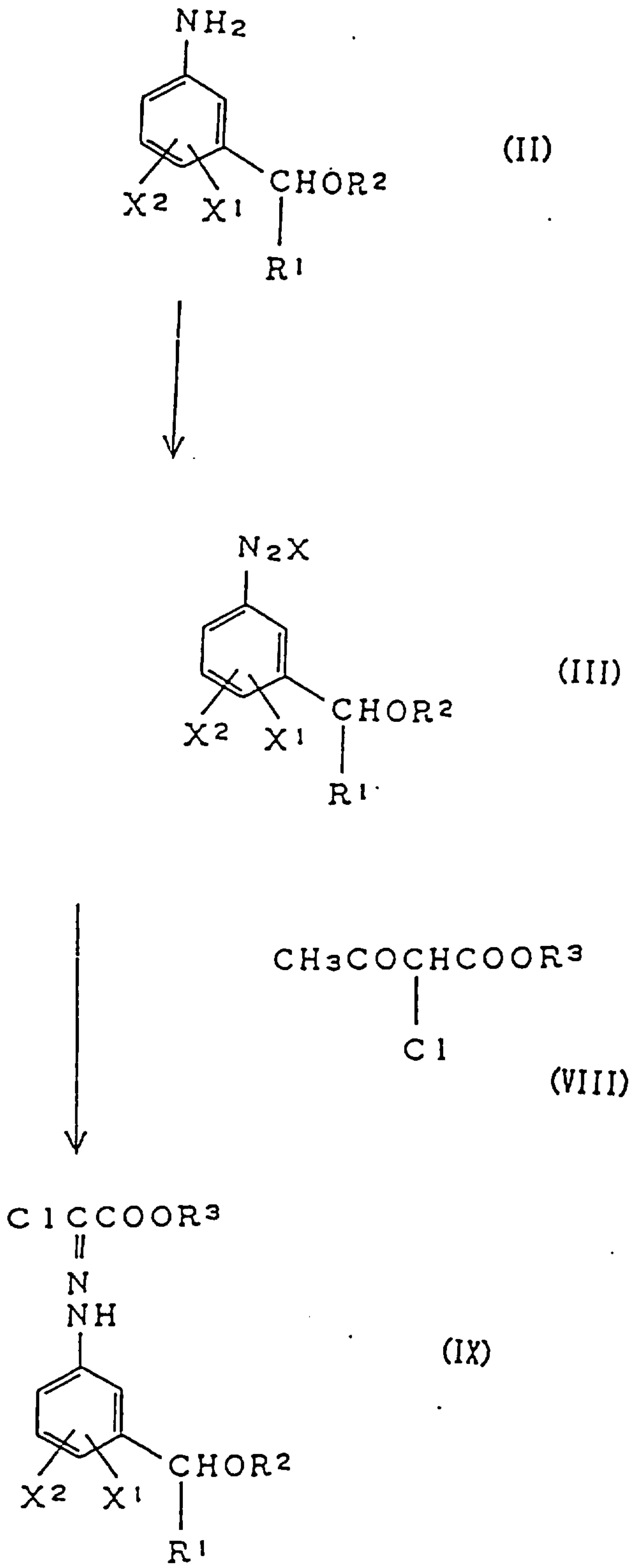

(II)

(III)

(VIII)

(IX)

wherein $R^1$, $R^2$, $X^1$, $X^2$, and X are as defined above, and $R^3$ represents a $C_1$-$C_4$ alkyl group.

(Condensation-ring closing reaction and auto-oxidation reaction)

As shown in the reaction formula below, oxamide derivative of formula (X) and benzaldehyde derivative of formula (XI) are reacted in a lower fatty acid such as acetic acid, propionic acid, or the like to obtain dihydrotriazolecarboxamide of formula (XII), followed by auto-oxidation of the dihydrotriazolecarboxamide. A condensation-ring closing reaction

takes place along with the auto-oxidation reaction at a temperature of 10 to 40°C and reaction time for 10 to 30 hours to produce a derivative of 1,5-diphenyl-1H-1,2,4-triazole-3-carboxamide of formula (I)

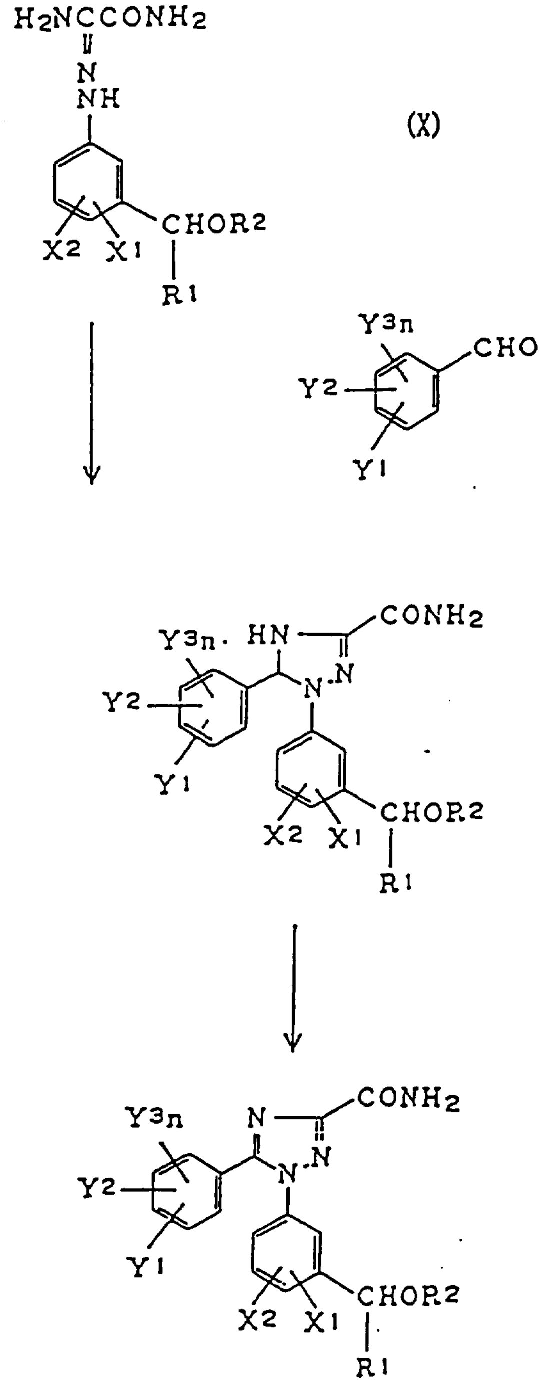

(X)

(XI)

(XII)

(I)

When the derivative of 1,5-diphenyl-1H-1,2,4-triazole-3-carboxamide of formula (I) manufactured by Process (A) or Process (B) is used as an antifungal agent, the compound is usually made into a formulation, together with adjuvants, in the form of powder, wettable or water-dispersible powder, granules, emulsion concentrate, or the like, although the compound may be used as is.

Such a formulation may contain one or more compounds of the present invention in an amount of 0.1 to 95%, preferably 0.5 to 90%, and more preferably 2 to 70% by weight.

Examples of carriers, diluents, surfactants, and the like which can be used as adjuvants in the composition of the present invention are as follows. talc, kaolin, bentonite, diatomaceous earth, white carbon, clay, and the like, can be used as solid carriers; and water, xylene, toluene, chlorobenzene, cyclohexane, cyclohexanone, dimethylsulfoxide, dimethylformamide, alcohols, and the like are used as diluents. Different types of surfactants may be used depending

on the effects intended; e.g., polyoxyethylene alkylaryl ether, polyoxyethylene sorbitan monolaurate, etc., as emulsifier; lignin sulfonate, dibutylnaphthalene sulfonate, etc., as dispersant; alkyl sulfonate, alkylphenyl sulfonate, etc., as wetting agents.

Depending on the types the formulations are used directly or after having been diluted to a prescribed concentration by diluents such as water.

When the formulation is used after dilution, its concentration is preferably in the range of 0.001 to 1.0%.

The compound of the present invention may be applied to fields, orchards, greenhouses, etc., in an amount of 20 to 5,000 g, preferably 50 to 1,000 g, per hectare.

The concentrations and amounts of use may be varied depending on types of the preparation, the time at which it is applied, the manner and the site in which it is used, and the plant to which it is applied. Thus, the amount and concentration may be increased or decreased without regard to the above specified range.

In addition, the compound of the present invention can be used in combination with other effective ingredients such as, for example, antifungals, antibacterials, insecticides, acaricides, or herbicides.

Furthermore, derivatives of 1,5-diphenyl-1H-1,2,4-triazole-3-carboxamide of formula (I) of the present invention are novel compounds and effective as antibacterial agents, especially as antifungal agents for gray mold.

Other features of the invention will become apparent in the course of the following description of exemplary embodiments which are given for illustration of the invention and are not intended to be limiting thereof.

## EXAMPLES

### Synthetic Example 1

<Synthesis of 1-[3-(1-butoxyethyl)phenyl]-5-phenyl-1H-1,2,4-triazole-3-carboxamide: Compound 36, $R^1$=$CH_3$, $R^2$=n-$C_4H_9$, $X^1$=$X^2$=H, $Y^1$=$Y^2$=$Y^3$=H, and n=1 in formula (I)]

<Synthesis by Process (A)>

#### Diazotization Reaction

18 ml of acetic acid, 6.72 g (34.8 mmol) of 3-(1-butoxyethyl)aniline [$R^1$=$CH_3$, $R^2$=n-$C_2H_9$, $X^1$=$X^2$=H in formula (II)], and 8 ml (90.2 mmol) of concentrated hydrochloric acid were charged into a 50 ml conical flask and cooled in an ice water bath. To the mixture was added dropwise an aqueous solution of 2.6 g (37.7 mmol) of $NaNO_2$ dissolved in 5 ml of water to produce 3-(l-butoxyethyl)benzene diazonium chloride [$R^1$=$CH_3$, $R^2$=n-$C_4H_9$, $X^1$=$X^2$=H, X=Cl in formula (III)].

#### Diazo Coupling Reaction

10 g (55.8 mmol) of hippuric acid [$X^1$=$X^2$=H, $Y^1$=$Y^2$=$Y^3$=H, and n=1 in formula (IV)] and 30 ml of acetic anhydride were charged into a 200 ml reaction flask and heated to dissolve hippuric acid. After quenching the solution to 10°C, 6.5 g (79.2 mmol) of sodium acetate was added, followed by dropwise addition of the diazonium chloride solution prepared above. The mixture was reacted for 30 minutes under ice-cooling for 2 hours at room temperature. Water was added to the reaction mixture to collect the diazo coupling reaction product by filtration. The filter cake was washed with water and dried in air to quantitatively obtain yellowish brown crystals of 2-phenyl-4,5-oxazoledione 4-[3-(1-butoxyethyl)phenyl]hydrazone [$R^1$=$CH_3$, $R^2$=n-$C_2H_9$, $X^1$=$X^2$=H, $Y^1$=$Y^2$=$Y^3$=H, and n=1 in formula (VI)].

#### Ammonolysis and Cyclocondensation reaction

25 ml of acetone and 6.9 g (18.9 mmol) of 2-phenyl-4,5-oxazoledione 4-[3-(1-butoxyethyl)phenyl] hydrazone [$R^1$=$CH_3$, $R^2$=n-$C_2H_9$, $X^1$=$X^2$=H, $Y^1$=$Y^2$=$Y^3$=H, and n=1 in formula (VI)] were charged into a 50 ml eggplant type flask. After the addition of 3.3 ml (48.7 mmol) of 28% aqueous ammonia, the mixture was stirred for 30 minutes. Then, 2.5 ml of 35% hydrochloric acid was added dropwise, followed by refluxing for 30 minutes to effect ring-closing reaction. The resultant reaction liquid was concentrated by evaporation, washed with water, and dried in air. This product was washed with a 1:1 mixture of ethyl acetate and n-hexane to obtain 5.2 g (14.3 mmol) of white crystals of the target compound, 1-[3-(1-butoxyethyl)phenyl]-5-phenyl-1H-1,2,4-triazole-3-carboxamide: Compound 36, $R^1$=$CH_3$, $R^2$=n-$C_4H_9$, $X^1$=$X^2$=H, $Y^1$=$Y^2$=$Y^3$=H, and n=1 in formula (I)] at a yield of 75%.

Synthetic Example 2

Synthesis of 1-[3-[(1-butoxy-2-methyl)propyl]phenyl]-5-(2-fluorophenyl)-1H-1,2,4-triazole-3-carboxamide: Compound 58, $R^1$=i-$C_3H_7$, $R^2$=n-$C_4H_9$, $X^1$=$X^2$=H, $Y^1$=2F, $Y^2$=$Y^3$=H, and n=1 in formula (I)

<Synthesis by Process (B)>

Diazonation Reaction

50 ml of methanol, 22.1 g (99.8 mmol) of 3-[(1-butoxy-2-methyl)propyl]aniline [$R^1$=i-$C_3H_7$, $R^2$=n-$C_4H_9$, $X^1$=$X^2$=H in formula (II)], and 23.5 ml (264.8 mmol) of 35% hydrochloric acid were charged into a 500 ml conical flask and cooled on an ice water bath. To the mixture was added dropwise an aqueous solution of 7.3 g (105.8 mmol) of $NaNO_2$ dissolved in 10 ml of water to produce 3-[(1-butoxy-2-methyl)propyl]benzene diazonium chloride [$R^1$=i-$C_3H_7$, $R^2$=n-$C_4H_9$, $X^1$=$X^2$=H in formula (III)].

Japp-Klingemann Reaction

15 g (99.6 mmol) of methyl chloroacetoacetate [$R^3$=$CH_3$ in formula (VIII)], 50 ml of methanol, and 20 g (243.8 mmol) of sodium acetate were measured and charged into a 500 ml conical flask, and cooled on an ice water bath. After the dropwise addition of the diazonium chloride solution prepared above, the mixture was reacted for 1 hour. The ice water bath was dismantled and the reaction was continued for a further 2 hours at room temperature. The precipitated product was collected by filtration, washed with water, and dried in air to obtain 32.3 g (94.8 mmol) of methyl chloro[[3-[(1-butoxy-2-methyl)propyl]phenyl]hydrazono] acetate [$R^1$=i-$C_3H_7$, $R^2$=n-$C_4H_9$, $X^1$=$X^2$=H, $R^3$=$CH_3$ in formula (IX)] at a yield of 95.2%.

Ammonolysis

17 g (49.9 mmol) of methyl chloro[[3-[(1-butoxy-2-methyl)propyl]phenyl]hydrazono] acetate [$R^1$=i-$C_3H_7$, $R^2$=n-$C_4H_9$, $X^1$=$X^2$=H, $R^3$=$CH_3$ in formula (IX)] was weighed and charged into a 300 ml eggplant type flask. After the addition of 100 ml of ethanol containing 13% of ammonia, the flask was capped and allowed to stand still at room temperature for 24 hours. The reaction liquid thus obtained was concentrated and shaken thoroughly with the addition of ethyl acetate, washed with water, and dried to quantitatively obtain oxamide [3-[(1-butoxy-2-methyl)propyl]phenyl]hydrazone [$R^1$=i-$C_3H_7$, $R^2$=n-$C_4H_9$, $X^1$=$X^2$=H in formula (X)].

Cyclocondensation Reaction and Auto-oxidation Reaction

6.1 g (19.9 mmol) of oxamide [3-[(1-butoxy-2-methyl)propyl]phenyl]hydrazone [$R^1$=i-$C_3H_7$, $R^2$n=-$C_4H_9$, $X^1$=$X^2$=H in formula (X)] was weighed and charged into a 100 ml eggplant type flask, followed by the addition of 30 ml of acetic acid to dissolve. To the solution was added 2.5 g (20.1 mmol) of o-fluorobenzaldehyde [$Y^1$=2-F, $Y^2$=$Y^3$=H, n=1] and the mixture was allowed to stand at room temperature overnight. The resultant reaction liquid was concentrated, thoroughly shaken with the addition of ethyl acetate, washed with water, dried, and concentrated to produce a crude product. This crude product was purified by column chromatography using a 10:1 mixture of chloroform and acetone as a solvent to obtain 6.2 g (15.1 mmol) of the title compound, 1-[3-[(1-butoxy-2-methyl)propyl]phenyl]-5-(2-fluorophenyl)-1H-1,2,4-triazole-3-carboxamide: Compound 58, $R^1$=i-$C_3H_7$, $R^2$=n-$C_4H_9$, $X^1$=$X^2$=H, $Y^1$=2F, $Y^2$=$Y^3$=H, and n=1 in formula (I)] at a yield of 75.9%.

The physicochemical characteristics of Compound 58 are shown in Table 2.

Synthetic Example 3

Synthesis of 3-(1-butoxyethyl)aniline [$R^1$=$CH_3$, $R^2$=n-$C_4H_9$, $X^1$=$X^2$=H in formula (II)]

a) Synthesis of $\alpha$-methyl-3-nitrobenzenemethanol [$R^1$=$CH_3$, $X^1$=$X^2$=H in formula (XVI)]

33 g (199.8 mmol) of 1-(3-nitrophenyl)ethanone [$R^1$=$CH_3$, $X^1$=$X^2$=H in formula (XV)] was weighed and charged into a 500 ml eggplant type flask, followed by the addition of 120 ml of ethanol. After cooling on an ice water bath, 4.2 g (111 mmol) of $NaBH_4$ was added in portions and the mixture was reacted for 30 minutes under ice cooling and for 2 hours at room temperature. The resulting reaction liquid was concentrated by evaporator and the residue was washed with water to quantitatively obtain light brown oil of $\alpha$-methyl-3-nitrobenzene methanol [$R^1$=$CH_3$, $X^1$=$X^2$=H in formula

(XVI)].

b) Synthesis of 1-(1-butoxyethyl)-3-nitrobenzene [$R^1$=$CH_3$, $R^2$=n-$C_4H_9$, $X^1$=$X^2$=H in formula (XIX)]

30 ml of n-hexane was charged into a 300 ml reaction flask, followed by the addition of 4.4 g (110 mmol) of 60% oily NaH. The mixture was thoroughly shaken to remove n-hexane by decantation. 30 ml of n-hexane was added to repeat the above procedure to remove oily component of NaH. 100 ml of DMF was added under ice cooling. To this was added 16.7 g (99.9 mmol) of α-methyl-3-nitrobenzene methanol [$R^1$=$CH_3$, $X^1$=$X^2$=H in formula (XVII)] in portions to convert it into Na-oxide. 16.4 g (119.7 mmol) of 1-bromo-n-butane [$R^2$=n-$C_4H_9$, $Z^2$=Br in formula (XVIII)] was added dropwise and reacted for 30 minutes under ice cooling and for 2 hours at room temperature. The resulting reaction mixture was poured into ice water and extracted with ethyl acetate to obtain 20.5 g of a crude product. This crude product was purified by column chromatography using a 5:1 mixture of n-hexane and ethyl acetate as a solvent to obtain 17.6 g (78.8 mmol) of light brown oily product of 1-(1-butoxyethyl)-3-nitrobenzene [$R^1$=$CH_3$, $R^2$=n-$C_4H_9$, $X^1$=$X^2$=H in formula (XIX)] at a. yield of 78.9%.

c) Synthesis of 3-(1-butoxyethyl)aniline [$R^1$=$CH_3$, $R^2$=n-$C_4H_9$, $X^1$=$X^2$=H in formula (II)]

11.7 g (52.4 mmol) of 1-(1-butoxyethyl)-3-nitrobenzene [$R^1$=$CH_3$, $R^2$=n-$C_4H_9$, $X^1$=$X^2$=H in formula (XIX)], 60 ml of ethanol, 1.2 g of activated charcoal, 0.3 g of $FeCl_3.6H_20$, and 1 ml of hydrazine hydrate were charged into a 300 ml reaction flask. After refluxing for 15 minutes, 12 ml of hydrazine hydrate was added dropwise over 30 minutes, followed by refluxing for a further 3 hours. The reaction liquid was aspirated through a filter paper to remove activated charcoal, and the filtrate was concentrated by evaporator and dissolved into ethyl acetate to obtain 9.1 g (47.1 mmol) of 3-(butoxyethyl)aniline [$R^1$=$CH_3$, $R^2$=n-$C_4H_9$, $X^1$=$X^2$=H in formula (II)] as a light brown oil at a yield of 89.8%.

Synthetic Example 4

Synthesis of 3-[(1-butoxy-2-methyl)propyl]aniline [$R^1$=i-$C_3H_7$, $R^2$=n-$C_4H_9$, $X^1$=$X^2$=H in formula (II)]

a) Synthesis of 2-methyl-1-phenyl propanone [$R^1$=i-$C_3H_7$, $X^1$=$X^2$=H in formula (XV)]

250 ml of dichloromethane was charged into a 1 1 reaction flask, followed by the addition of 67 g (502.5 mmol) of $AlCl_3$. After cooling the mixture on an ice water bath to 5°C, 53.3 g (500.2 mmol) of isobutyryl chloride [$R^1$=$C_3H_7$, X=Cl in formula (XIV)] was added dropwise.

Then, 39.06 g (500 mmol) of benzene was added dropwise, while maintaining the temperature at 5°C and the mixture was reacted for 2 hours at 5°C and 1 hour at 10°C, followed by refluxing for 1 hour. The resulting reaction mixture was poured into ice water, washed with water, and dried to obtain a concentrated crude product. This crude product was distilled under vacuum to obtain 64.7 g (436.5 mmol) of 2-methyl-1-phenylpropanone [$R^1$=i-$C_3H_7$, $X^1$=$X^2$=H in formula (XV)] as colorless oil at a. yield of 87.3%.

b) Synthesis of 2-methyl-1-(3-nitrophenyl) propanone [$R^1$=i-$C_3H_7$, $X^1$=$X^2$=H in formula (XVI)]

150 ml of 95% sulfuric acid was charge into a 500 ml reaction flask and cooled to -20°C in a dry ice-acetone bath. 15 ml of a concentrated nitric acid (61%, d=1.38) was added dropwise in portions to it and the mixture was cooled to -20°C. To this was added dropwise in portions 29.6 g (199.7 mmol) of 2-methyl-1-phenyl propanone [$R^1$=i-$C_3H_7$, $X^1$=$X^2$=H in formula (XV)] so as not to raise the temperature above -5°C. The mixture reacted for 1 hour at -5 to -10°C. After the reaction the resulting reaction mixture was poured into ice water, extracted with ethyl acetate, washed with water, and dried to concentrate, thus obtaining 38.0 g of a light brown oily product of 2-methyl-1-(3-nitrophenyl) propanone [$R^1$=i-$C_3H_7$, $X^1$=$X^2$=H in formula (XVI)] at a yield of 98.5%.

c) Synthesis of α-(1-methylethyl)-3-nitrobenzenemethanol [$R^1$=i-$C_3H_7$, $X^1$=$X^2$=H in formula (XVI)]

33 g (170.8 mmol) of 2-methyl-1-(3-nitrophenyl) propanone [$R^1$=i-$C_3H_7$, $X^1$=$X^2$=H in formula (XVI)] was charged into a 500 ml eggplant type flask. 100 ml of ethanol was added and the mixture was cooled on an ice water bath. To this, 4.2 g (111 mmol) of $NaBH_4$ was slowly added and the mixture was reacted for 30 minutes under ice cooling and for 2 hours at room temperature. The resulting reaction liquid was concentrated by evaporator, and the residue was washed with water to quantitatively obtain α-(1-methylethyl)-3-nitrobenzenemethanol [$R^1$=i-$C_3H_7$, $X^1$=$X^2$=H in formula (XVI)] as a light brown oily product.

d) Synthesis of 1-[(1-butoxy-2-methyl)propyl]-3-nitrobenzene [$R^1$=i-$C_3H_7$, $R^2$=n-$C_4H_9$, $X^1$=$X^2$=H in formula (XIX)]

30 ml of n-hexane was charged in a 300 ml reaction flask, followed by the addition of 4.4 g (110 mmol) of 60% oily NaH. The mixture was thoroughly shaken to remove n-hexane by decantation. 30 ml of n-hexane was added to repeat the above procedure to remove oily component of NaH. 100 ml of DMF was added under ice cooling. To this was added 19.5 g (99.9 mmol) of $\alpha$-(1-methylethyl)-3-nitrobenzene methanol [$R^1$=i-$C_3H_7$, $X^1$=$X^2$=H in formula (XVI)] in portions to convert it into Na-oxide. 20.6 g (150.3 mmol) of 1-bromo-n-butane was added dropwise and reacted for 30 minutes under ice cooling and for 2 hours at room temperature. The resulting reaction mixture was poured into ice water and extracted with ethyl acetate to obtain 23 g of a crude product. This crude product was purified by column chromatography using a 5:1 mixture of n-hexane and ethyl acetate as a solvent to obtain 18.5 g (73.6 mmol) of light brown oily product of 1-[(1-butoxy-2-methyl)propyl]-3-nitrobenzene [$R^1$=i-$C_3H_7$, $R^2$=n-$C_4H_9$, $X^1$=$X^2$=H in formula (XIX)] at a yield of 73.7%.

e) Synthesis of 3-[(1-butoxy-2-methyl)propyl]aniline [$R^1$=i-$C_3H_7$, $R^2$=n-$C_4H_9$, $X^1$=$X^2$=H in formula (II)]

12.5 g (49.7 mmol) of 1-[(1-butoxy-2-methyl)propyl]-3-nitrobenzene [$R^1$=i-$C_3H_7$, $R^2$=n-$C_4H_9$, $X^1$=$X^2$=H in formula (XIX)], 60 ml of ethanol, 1.2 g of activated charcoal, 0.3 g of $FeCl_3 \cdot 6H_2O$, and 1 ml of hydrazine hydrate were charged into a 300 ml reaction flask. After refluxing for 15 minutes, 12 ml of hydrazine hydrate was added dropwise over 30 minutes, followed by refluxing for a further 3 hours. The reaction liquid was aspirated through a filter paper to remove activated charcoal, and the residue was concentrated by evaporator and dissolved into ethyl acetate, to obtain 10 g of crude product. This crude product was purified by column chromatography using a 10:1 mixture of n-hexane and ethyl acetate as a solvent to obtain 8.5 g (38.4 mmol) of light yellow oily product of 3-[(1-butoxy-2-methyl)propyl]aniline [$R^1$=i-$C_3H_7$, $R^2$=n-$C_4H_9$, $X^1$=$X^2$=H in formula (II)] at a yield of 77.3%.

Formulation Example 1 <Dust>

| | parts by weight |
|---|---|
| Compound 1 | 3 |
| Clay | 40 |
| Talc | 57 |

The above ingredients were pulverized and mixed to obtain a dust.

Preparation Example 2 <Wettable Powder>

| | parts by weight |
|---|---|
| Compound 40 | 50 |
| Lignin sulfonate | 5 |
| Alkyl sulfonate | 3 |
| Diatomaceous earth | 42 |

The above ingredients were pulverized and mixed to obtain a wettable powder.

Preparation Example 3 <Granules>

| | parts by weight |
|---|---|
| Compound 3 | 5 |
| Bentonite | 43 |
| Clay | 45 |
| Lignin sulfonate | 7 |

The above ingredients were mixed and kneaded with the addition of water. The mixture was extruded and then dried to obtain granules.

Preparation Example 4

<Emulsion Concentrate>

| | parts by weight |
|---|---|
| Compound 84 | 20 |
| Polyoxyethylene alkylaryl ether | 10 |
| Polyoxyethylenesorbitan monolaurate | 3 |
| Xylene | 67 |

The above ingredients were mixed and dissolved to obtain an emulsion concentrate.

**Test Example 1**

<Test for controlling effect against cucumber gray mold (Botrytis cinerea on cucumber)>

A wettable powder similar to that prepared in Preparation Example 2, diluted with water to a prescribed concentration, was sprayed onto the cucumber plants of the second-leaf stage (variety: *SAGAMI HAMPAKU*, one plant per pot and 3 pots being used in the treated plot) growing in a unglazed pots(diameter: 10 cm), in an amount of 5 ml per pot. After drying the sprayed leaves in the air, a circular cutting (diameter: 4 mm) of agar containing the fungi of Botrytis cinerea, which had been cultured in advance in a potato-sucrose agar medium at 20°C for 3 days, was attached directly to the center part of the leaves, and the pots maintained at 20-22°C under high humidity conditions. Three days after the inoculation, lesion areas of the disease were examined to calculate the inhibition rate according to the following equation

$$\text{Inhibition Rate (\%)} = \left[ 1 - \frac{\text{Lesion area in treated plot}}{\text{Lesion area in untreated plot}} \right] \times 100$$

The results are shown in Table 3.

**Test Example 2**

<Test for controlling effect against cucumber downy mildew *(Pseudoperonospora cubensis* on cucumber)>

A wettable powder similar to that prepared in Preparation Example 2, diluted with water to a prescribed concentration, was sprayed onto the cucumber plants of the second-leaf stage (variety: *SAGAMI* HAMPAKU, one plant per pot and 3 pots being used in the treated plot) growing in unglazed pots(diameter: 10 cm), in an amount of 5 ml per pot. After drying the sprayed leaves in the air, a suspension of the spores of *Pseudoperonospore cubensis,* which had been collected from the contracted leaf of cucumber, was sprayed onto the leaves, and the pots were maintained at 20-22°C under high humidity conditions for 24 hours; 5-7 days after the inoculation, lesion areas of the disease were examined to calculate the inhibition rate according to the above equation.

The results are shown in Table 3.

**Test Example 3**

<Test for controlling effect against tomato late blight (*Phytophthora infestans* on tomato)>

A wettable powder similar to that prepared in Preparation Example 2, diluted with water to a prescribed concentration, was sprayed onto the young seedlings of tomato of the third leaf stage (variety: *FUKUJU* No. 2, one plant per pot and 3 pots being used in the treated plot) growing in unglazed pots(diameter: 10 cm), in an amount of 5 ml per pot.

After drying the sprayed leaves in the air, a suspension of the spores of *Phytophthora infestans*, which had been collected from the contracted leaf of tomato, was sprayed onto the leaves, and the pots were maintained at 20-22°C under high humidity conditions for 24 hours, followed by growing in a greenhouse; 5-7 days after the inoculation, lesion areas of the disease were examined to calculate the inhibition rate according to the above equation.

The results are shown in Table 3.

**Test Example 4**

<Test for controlling effect against wheat red rust *(Puccinia recondita* on wheat)>

A wettable powder similar to that prepared in Preparation Example 2, diluted with water to a prescribed concentration, was sprayed onto the young seedlings of wheat of the second leaf stage (variety: *NORIN* No. 64, 16 seedlings per pot and 3 pots being used in the treated plot) growing in unglazed pots(diameter: 10 cm), in an amount of 5 ml per pot. After drying the sprayed leaves in the air, a suspension of the summer spores of *Puccinia recondita*, which had been collected from the contracted leaf of wheat, was sprayed onto the leaves, and the pots were maintained at 20-23°C under high humidity conditions for 24 hours, followed by growing in a greenhouse; 7-10 days after the inoculation, lesion areas of the disease were examined to calculate the inhibition rate according to the above equation.

The results are shown in Table 3.

**Test Example 5**

<Test for controlling effect against wheat powdery mildew *(Erysiphe graminis f. sp. tritici* on wheat)>

A wettable powder similar to that prepared in Preparation Example 2, diluted with water to a prescribed concentration, was sprayed onto the young seedlings of wheat of the second leaf stage (variety: *NORIN* No. 64, 16 seedlings per pot and 3 pots being used in the treated plot) growing in unglazed pots (diameter: 10 cm), in an amount of 5 ml per pot. After drying the sprayed leaves in the air, a suspension of the summer spores of *Erysiphe graminis f. sp. tritici*, which had been collected from the attacked leaf of wheat, was sprayed onto the leaves, and the pots were maintained at 20-24°C under high humidity conditions for 24 hours, followed by growing in a greenhouse; 9-11 days after the inoculation, lesion areas of the disease were examined to calculate the inhibition rate according to the above equation.

The results are shown in Table 3.

TABLE 3

| Compound No. | Inhibition rate (%) at concentration of 500 ppm | | | | |
|---|---|---|---|---|---|
| | Cucumber gray mold | Cucumber downy mildew | Tomato late blight | Wheat leaf rust | Wheat powdery mildew |
| 1 | 90 | 63 | | | 90 |
| 2 | 70 | | | | |
| 3 | 90 | | | | |
| 4 | 72 | 70 | | | |
| 5 | 60 | | | | |
| 7 | 84 | | | | |
| 8 | 98 | | | | |
| 10 | 84 | | | | |
| 11 | 90 | | | | |
| 12 | 94 | | | | |
| 13 | 98 | | | | |
| 14 | 80 | | | | |
| 15 | 88 | | | | |
| 18 | 74 | | | | |
| 22 | 64 | | | | |
| 23 | 76 | | | | |
| 24 | 93 | | | | |
| 25 | 94 | | | | |
| 26 | 90 | | | | |
| 27 | 88 | | | | |
| 28 | 90 | | | | |

TABLE 3 (Continued)

| Compound No. | Inhibition rate (%) at concentration of 500 ppm | | | | |
| --- | --- | --- | --- | --- | --- |
| | Cucumber gray mold | Cucumber downy mildew | Tomato late blight | Wheat leaf rust | Wheat powdery mildew |
| 29 | 70 | | | | |
| 33 | 68 | | | | |
| 34 | 84 | | | | |
| 35 | 82 | | | | |
| 36 | 66 | | | | |
| 37 | 70 | | | | |
| 38 | 64 | | 64 | | |
| 40 | | | | 95 | 80 |
| 43 | 93 | | | | |
| 44 | 62 | | | | |
| 45 | 94 | | | | |
| 46 | 84 | | | | |
| 50 | 74 | | | | |
| 51 | 90 | | | | |
| 53 | 94 | | | | |
| 54 | 94 | | | | |
| 56 | 94 | | | | |
| 57 | 90 | | | | |
| 58 | 84 | | | | |
| 59 | 90 | | | | |
| 60 | 93 | | | | |

TABLE 3 (Continued)

| Compound No. | Inhibition rate (%) at concentration of 500 ppm | | | | |
|---|---|---|---|---|---|
| | Cucumber gray mold | Cucumber downy mildew | Tomato late blight | Wheat leaf rust | Wheat powdery mildew |
| 61 | 90 | | | | |
| 62 | 78 | | | | |
| 63 | 90 | | | | |
| 65 | 88 | | | | |
| 66 | 74 | | | | |
| 67 | 94 | | | | |
| 68 | 80 | | | | |
| 69 | 80 | | | | |
| 70 | 70 | | | | |
| 71 | 84 | | | | |
| 72 | 94 | | | | |
| 73 | 60 | | | | |
| 75 | 88 | | | | |
| 78 | 68 | | | | |
| 79 | 87 | | | | |
| 80 | 92 | | | | |
| 81 | 93 | | | | |
| 82 | 79 | 60 | | | |
| 84 | 95 | | | | |
| 85 | 60 | | | | |
| 86 | 74 | | | | |

**Claims**

1. A derivative of 1,5-diphenyl-1H-1,2,4-triazole-3-carboxamide represented by the following general formula (I),

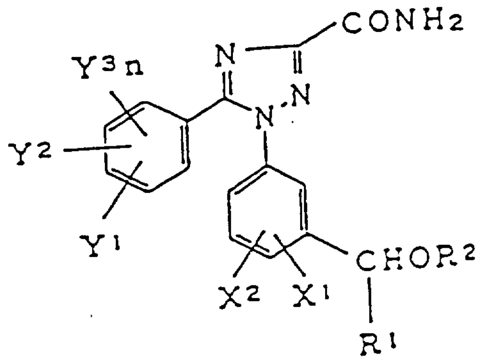

(I)

wherein $R^1$ is a $C_1$-$C_6$ alkyl group, $C_3$-$C_6$ cycloalkyl group, $C_1$-$C_5$ fluoroalkyl group, ($C_1$-$C_4$ alkoxy)methyl group, or phenyl group, $R^2$ is a $C_1$-$C_8$ alkyl group, ($C_3$-$C_6$ cycloalkyl)methyl group, $C_2$-$C_5$ fluoroalkyl group, ($C_1$-$C_4$ alkoxy) ($C_1$-$C_4$ alkyl) group, phenyl group, phenylmethyl group, or phenylmethyl group substituted by a $C_1$-$C_4$ alkyl group or a halogen atom, $X^1$ is a hydrogen atom, a $C_1$-$C_4$ alkyl group, a $C_1$-$C_4$ alkoxy group, or a halogen atom, $X^2$ is a hydrogen atom, a $C_1$-$C_4$ alkyl group, or a halogen atom, $Y^1$ is a hydrogen atom, a halogen atom, a $C_1$-$C_4$ alkyl group, a $C_1$-$C_4$ alkoxy group, a $C_1$-$C_4$ fluoroalkoxy group, a HO group, a HOOC group, or a ($C_1$-$C_4$ alkoxy) carbonyl group, $Y^2$ is a hydrogen atom, a $C_1$-$C_4$ alkyl group, or a halogen atom, $Y^3$ is a hydrogen atom or a halogen atom, and n is 1 or 2.

2. A derivative of 1,5-diphenyl-1H-1,2,4-triazole-3-carboxamide according to Claim 1 wherein
$R^1$ is a $C_1$-$C_3$ alkyl group, $R^2$ is a $C_1$-$C_5$ alkyl group, $X^1$ is a hydrogen atom or a $C_1$ or $C_2$ alkyl group, $X^2$ is a hydrogen atom, $Y^1$ is a hydrogen atom or a fluorine atom, $Y^2$ is a hydrogen or fluorine atom, and $Y^3$ is a hydrogen atom.

3. A compound according to claim 1 which is

1-[3-(1-butoxyethyl)phenyl]-5-phenyl-1H-1,2,4-triazole-3-carboxamide;
1-[3-[(1-butoxy-2-methyl)propyl]phenyl]-5-(2-fluorophenyl)-1H-1,2,4-triazole-3-carboxamide;
1-[3-[(1-butoxy-2-methyl)propyl]-6-methyl]phenyl]-5-(2,3-difluorophenyl)-1H-1,2,4-triazole-3-carboxamide
1-[3-[(1-butoxy-2-methyl)propyl]-6-methyl]phenyl]5-(2-fluorophenyl)-1H-1,2, 4-triazole-3-carboxamide ; or
1-[3-[(1-butoxy-2-methyl)propyl]-6-methyl]phenyl]5-phenyl-1H-1,2,4-triazole-3-carboxamide.

4. A process for the manufacture of 1,5-diphenyl-1H-1,2,4-triazole-3-carboxamide derivative of following formula (I),

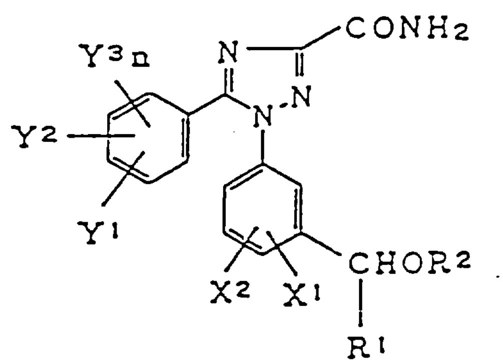

(I)

wherein $R^1$, $R^2$, $X^1$, $X^2$, $Y^1$, $Y^2$, $Y^3$, and n are as defined in Claim 1, which comprises:

reacting oxazoledione hydrazone derivative (VI) and ammonia to obtain a hippuric acid amide derivative (VII) according to the following reaction formula:

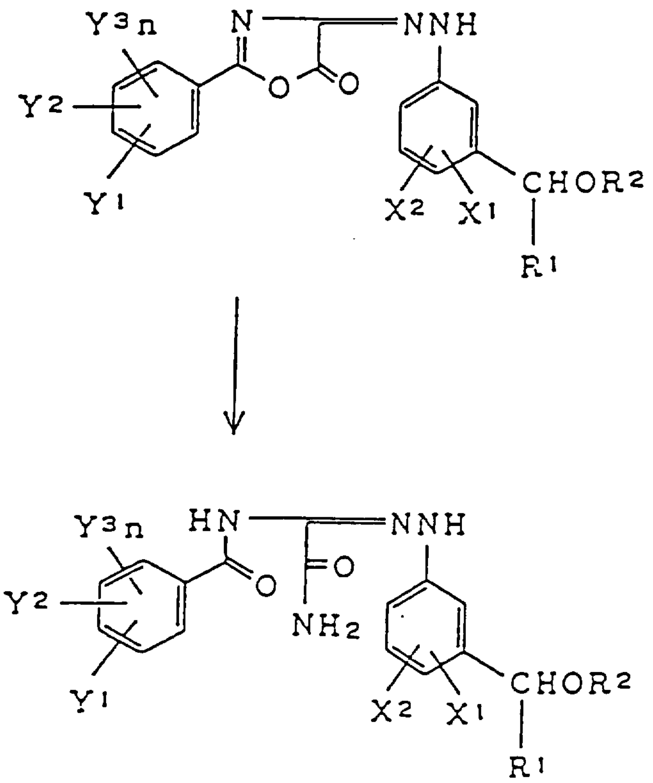

(VI)

(VII)

and

subjecting said derivative of hippuric acid amide to a cyclocondensation reaction.

**5.** A process for the manufacture of 1,5-diphenyl-1H-1,2,4-triazole-3-carboxamide derivative of following general formula (I),

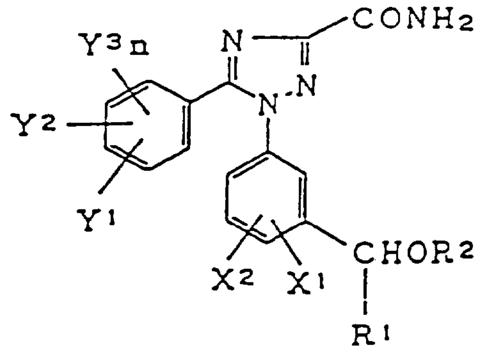

(I)

wherein $R^1$, $R^2$, $X^1$, $X^2$, $Y^1$, $Y^2$, $Y^3$, and n are as defined in Claim 1, which comprises:

reacting oxamide derivative of formula (X) and benzaldehyde derivative of formula (XI) to produce dihydrotriazolecarboxamide of formula (XII) according to the following reaction formula:

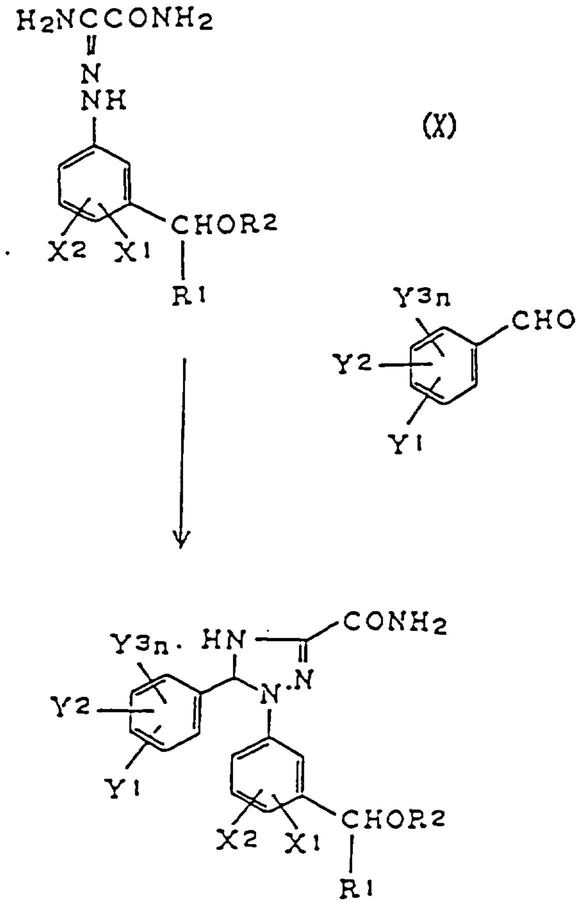

(X)

(XI)

(XII)

and
oxidizing the dihydrotriazolecarboxamide.

6. An antimicrobial composition comprising as an effective component at least one 1,5-diphenyl-1H-1,2,4-triazole-3-carboxamide derivative according to any of claims 1 to 3.

7. An antimicrobial composition according to Claim 6 which is an antimicrobial composition against gray mold.

8. A method of making an antimicrobial composition which comprises mixing at least one compound according to any of claims 1 to 3 with agricultural or horticultural carrier.

9. A method for the antimicrobial treatment of an agricultural or horticultural plot or crop which comprises applying to the plot or crop at least one compound according to any of claims 1 to 3.

10. A method according to claim 9 for the prevention or treatment of one or more gray mold, downy mildew, late blight, leaf rust and powdery mildew.

**Patentansprüche**

1. Derivat von 1,5-Diphenyl-1H-1,2,4-triazol-3-carboxamid, angegeben durch die folgende allgemeine Formel (I),

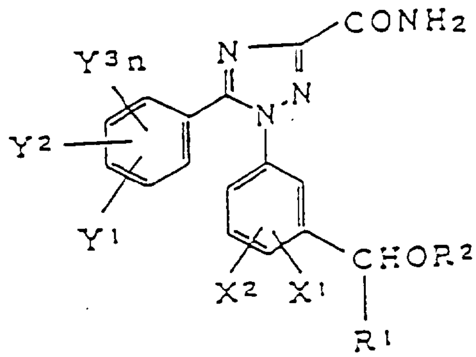

(I)

in der $R^1$ eine $C_1$-$C_6$-Alkylgruppe, $C_3$-$C_6$-Cycloalkylgruppe, $C_1$-$C_5$-Fluoralkylgruppe, ($C_1$-$C_4$-Alkoxy)methylgruppe oder Phenylgruppe bedeutet, $R^2$ eine $C_1$-$C_8$-Alkylgruppe, ($C_3$-$C_6$-Cycloalkyl)methylgruppe, $C_2$-$C_5$-Fluoralkylgruppe, ($C_1$-$C_4$-Alkoxy) ($C_1$-$C_4$-alkyl)gruppe, Phenylgruppe, Phenylmethylgruppe oder Phenylmethylgruppe, die durch eine $C_1$-$C_4$-Alkylgruppe oder ein Halogenatom substituiert ist, bedeutet, $X^1$ ein Wasserstoffatom, eine $C_1$-$C_4$-Alkylqruppe, eine $C_1$-$C_4$-Alkoxygruppe oder ein Halogenatom bedeutet, $X^2$ ein Wasserstoffatom, eine $C_1$-$C_4$-Alkylgruppe oder ein Halogenatom bedeutet, $Y^1$ ein Wasserstoffatom, ein Halogenatom, eine $C_1$-$C_4$-Alkylgruppe, eine $C_1$-$C_4$-Alkoxygruppe, eine $C_1$-$C_4$-Fluoralkoxygruppe oder eine HO-Gruppe, eine HOOC-Gruppe oder eine ($C_1$-$C_4$-Alkoxy)carbonylgruppe bedeutet, $Y^2$ ein Wasserstoffatom, eine $C_1$-$C_4$-Alkylgruppe oder ein Halogenatom bedeutet, $Y^3$ ein Wasserstoffatom oder ein Halogenatom bedeutet und n 1 oder 2 ist.

**2.** Derivat von 1,5-Diphenyl-1H-1,2,4-triazol-3-carboxamid nach Anspruch 1, wobei $R^1$ eine $C_1$-$C_3$-Alkylqruppe, $R^2$ eine $C_1$-$C_5$-Alkylgruppe, $X^1$ ein Wasserstoffatom oder eine $C_1$- oder $C_2$-Alkylqruppe, $X^2$ ein Wasserstoffatom, $Y^1$ ein Wasserstoffatom oder ein fluoratom, $Y^2$ ein Wasserstoff- oder Fluoratom und $Y^3$ ein Wasserstoffatom bedeuten.

**3.** Verbindung nach Anspruch 1, die

1-[3-(1-Butoxyethyl)phenyl]-5-phenyl-1H-1,2,4-triazol-3-carboxamid;
1-[3-[(1-Butoxy-2-methyl)propyl]phenyl]-5-(2-fluorphenyl)1H-1,2,4-triazol-3-carboxamid;
1-[3-[(1-Butoxy-2-methyl)propyl]-6-methyl]phenyl]-5-(2,3-difluorphenyl)-1H-1,2,4-triazol-3-carboxamid;
1-[3-[(1-Butoxy-2-methyl)propyl]-6-methyl]phenyl]-5-(2-fluorphenyl)-1H-1,2,4-triazol-3-carboxamid oder
1-[3-[(1-Butoxy-2-methyl)propyl]-6-methyl]phenyl]-5-phenyl1H-1,2,4-triazol-3-carboxamid ist.

**4.** Verfahren zur Herstellung eines 1,5-Diphenyl-1H-1,2,4-triazol-3-carboxamid-Derivats der folgenden Formel (I)

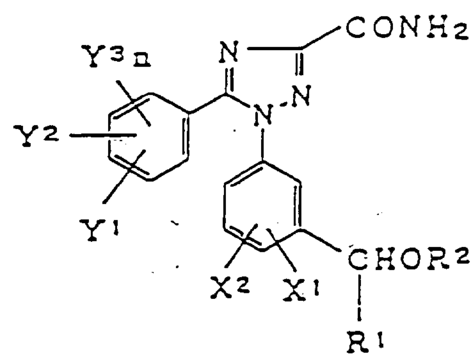

(I)

in der $R^1$, $R^2$, $X^1$, $X^2$, $Y^1$, $Y^2$, $Y^3$ und n wie in Anspruch 1 definiert sind, umfassend:
Umsetzung eines Oxazoldionhydrazon-Derivats (VI) mit Ammoniak unter Bildung eines Hippursäureamid-Derivats (VII), entsprechend der folgenden Reaktionsgleichung

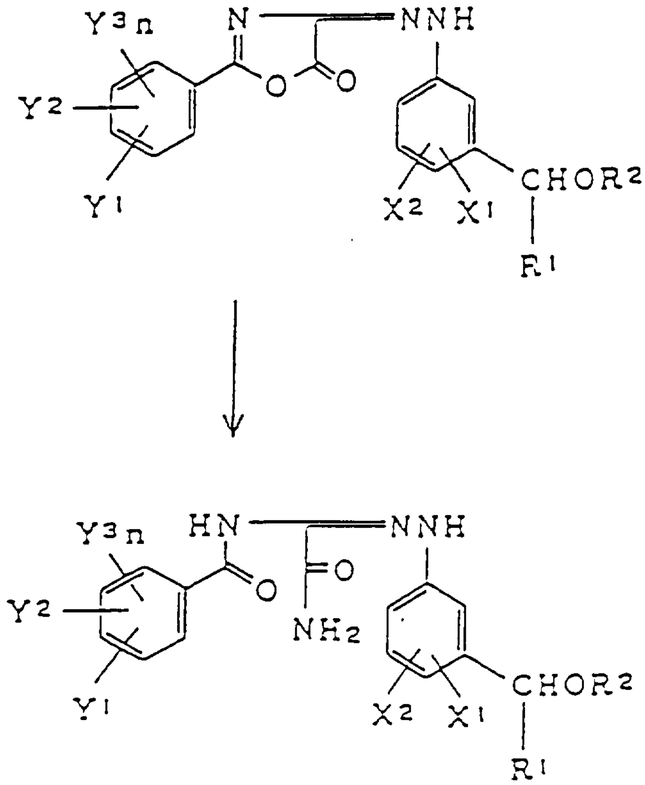

(VI)

(VII)

und Durchführung einer Cyclokondensationsrekation an dem Derivat von Hippursäureamid.

**5.** Verfahren zur Herstellung eines 1,5-Diphenyl-1H-1,2,4-triazol-3-carboxamid-Derivats der folgenden allgemeinen Formel (I),

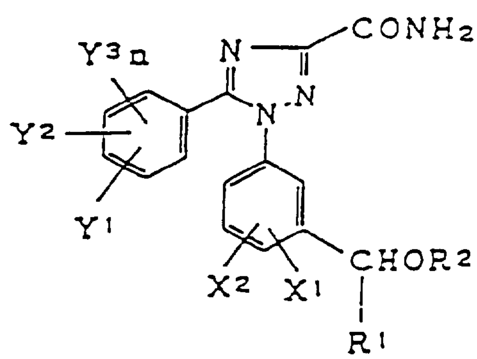

(I)

in der $R^1$, $R^2$, $X^1$, $X^2$, $Y^1$, $Y^2$, $Y^3$ und n wie in Anspruch 1 definiert sind, umfassend:
Umsetzung eines Oxamid-Derivats der Formel (X) mit einem Benzaldehyd-Derivat der Formel (XI) zur Bildung von Dihydrotriazolcarboxamid der Formel (XII) nach der folgenden Reaktionsgleichung:

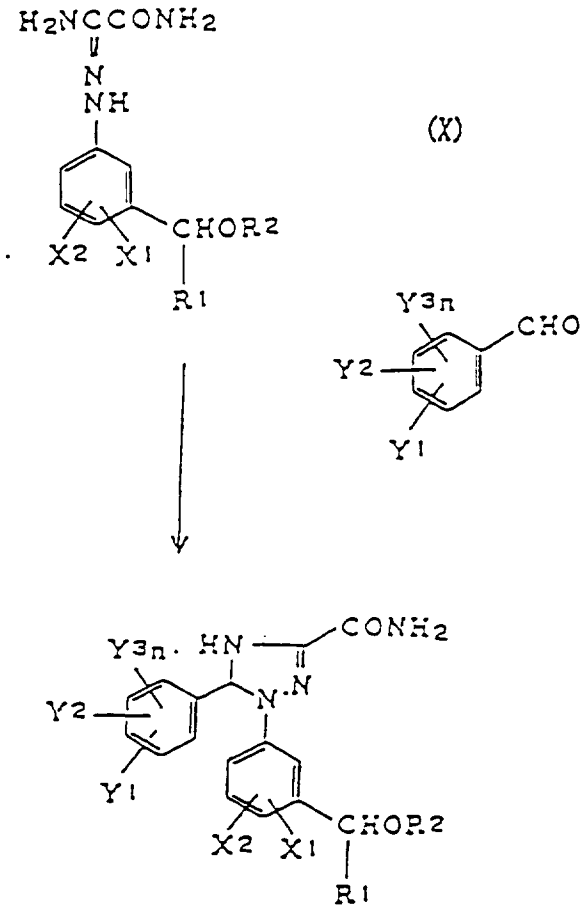

(X)

(XI)

(XII)

und Oxidieren des Dihydrotriazolcarboxamids.

6. Antimikrobielles Mittel, umfassend als Wirkstoff mindestens ein 1,5-Diphenyl-1H-1,2,4-triazol-3-carboxamid-Derivat nach einem der Ansprüche 1 bis 3.

7. Antimikrobielles Mittel nach Anspruch 6, das ein antimikrobielles Mittel gegen Grauschimmel ist.

8. Verfahren zur Herstellung eines antimikrobiellen Mittels, umfassend das Vermischen mindestens einer Verbindung nach einem der Ansprüche 1 bis 3 mit einem Träger für Landwirtschaft oder Gartenbau.

9. Verfahren zur antimikrobiellen Behandlung einer Stelle oder von Nutzpflanzen in der Landwirtschaft oder im Gartenbau, umfassend das Aufbringen mindestens einer Verbindung nach einem der Ansprüche 1 bis 3 auf die Stelle oder die Nutzpflanzen.

10. Verfahren nach Anspruch 9 zur Verhinderung oder Behandlung von Grauschimmel, falschem Mehltau, Braunfäule, Blattrost und echtem Mehltau.

**Revendications**

1. Un dérivé du 1,5-diphényl-1H-1,2,4-triazole-3-carboxamide représenté par la formule générale (I) suivante:

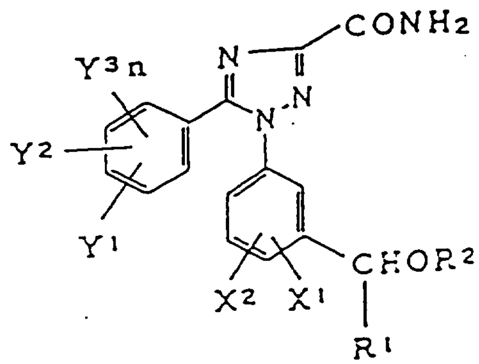

(I)

dans laquelle $R^1$ est un groupe alkyle en $C_1$-$C_6$, un groupe cycloalkyle en $C_3$-$C_6$, un groupe fluoroalkyle en $C_1$-$C_5$, un groupe (alcoxy en $C_1$-$C_4$)méthyle ou un groupe phényle, $R^2$ est un groupe alkyle en $C_1$-$C_8$, un groupe (cycloalkyle en $C_3$-$C_6$)méthyle, un groupe fluoroalkyle en $C_2$-$C_5$, un groupe (alcoxy en $C_1$-$C_4$)(alkyle en $C_1$-$C_4$), un groupe phényle, un groupe phénylméthyle ou un groupe phénylméthyle substitué par un groupe alkyle en $C_1$-$C_4$ ou un atome d'halogène, $X^1$ est un atome d'hydrogène, un groupe alkyle en $C_1$-$C_4$, un groupe alcoxy en $C_1$-$C_4$ ou un atome d'halogène, $X^2$ est un atome d'hydrogène, un groupe alkyle en $C_1$-$C_4$ ou un atome d'halogène, $Y^1$ est un atome d'hydrogène, un atome d'halogène, un groupe alkyle en $C_1$-$C_4$, un groupe alcoxy en $C_1$-$C_4$, un groupe fluoroalkyle en $C_1$-$C_4$, un groupe HO, un groupe HOOC ou un groupe (alcoxy en $C_1$-$C_4$)carbonyle, $Y^2$ est un atome d'hydrogène, un groupe alkyle en $C_1$-$C_4$ ou un atome d'halogène, $Y^3$ est un atome d'hydrogène ou un atome d'halogène et n est 1 ou 2.

**2.** Un dérivé du 1,5-diphényl-1H-1,2,4-triazole-3-carboxamide selon la revendication 1 dans lequel $R^1$ est un groupe alkyle en $C_1$-$C_3$, $R^2$ est un groupe alkyle en $C_1$-$C_5$, $X^1$ est un atome d'hydrogène ou un groupe alkyle en $C_1$ ou $C_2$, $X^2$ est un atome d'hydrogène, $Y^1$ est un atome d'hydrogène ou un atome de fluor, $Y^2$ est un atome d'hydrogène ou de fluor et $Y^3$ est un atome d'hydrogène.

**3.** Un composé selon la revendication 1 qui est

le 1-[3-(1-butoxyéthyl)phényl]-5-phényl-1H-1,2,4-triazole-3-carboxamide ;
le 1-[3-[(1-butoxy-2-méthyl)propyl]phényl]-5-(2-fluorophényl)-1H-1,2,4-triazole-3-carboxamide;
le 1-[3-[(1-butoxy-2-méthyl)propyl]-6-méthyl]phényl]-5-(2,3-difluorophényl)-1H-1,2,4-triazole-3-carboxamide;
le 1-[3-[(1-butoxy-2-méthyl)propyl]-6-méthyl]phényl]-5-(2-fluorophényl)-1H-1,2,4-triazole-3-carboxamide; ou
le 1-[3-[(1-butoxy-2-méthyl)propyl]-6-méthyl]phényl]-5-phényl-1H-1,2,4-triazole-3-carboxamide.

**4.** Un procédé pour la production d'un dérivé du 1,5-diphényl-1H-1,2,4-triazole-3-carboxamide de formule (I) suivante,

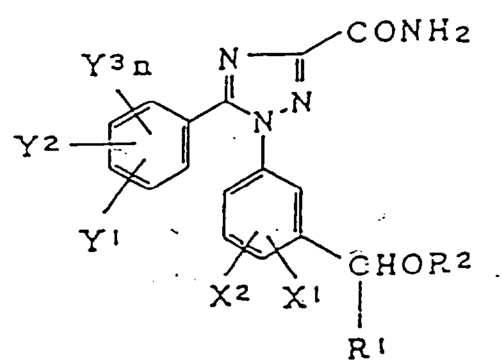

(I)

où $R^1$, $R^2$, $X^1$, $X^2$, $Y^1$, $Y^2$, $Y^3$ et n sont tels que définis dans la revendication 1, qui comprend :
la réaction d'un dérivé d'oxazoledione hydrazone (VI) et d'ammoniac pour obtenir un dérivé amide d'acide hippurique (VII) selon la formule de réaction suivante :

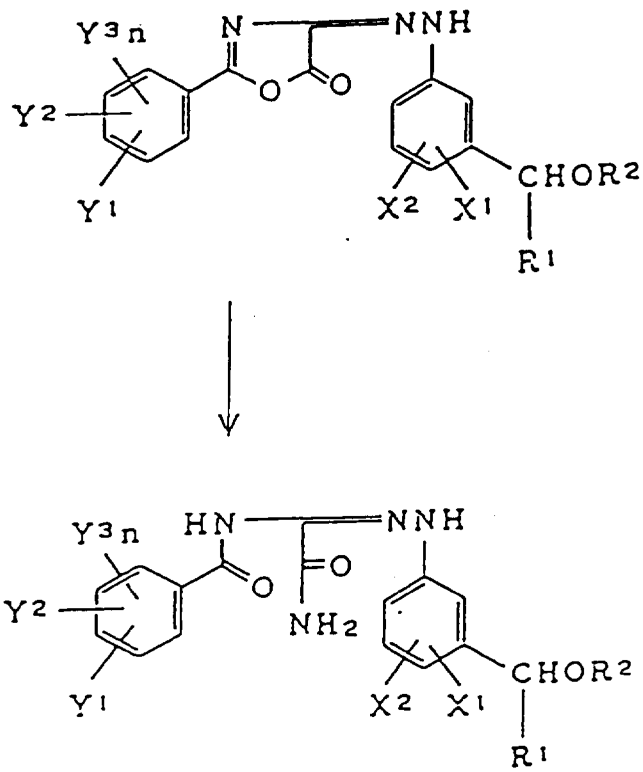

(VI)

(VII)

et la soumission dudit dérivé amide d'acide hippurique à une réaction de cyclocondensation.

**5.** Un procédé pour la production d'un dérivé du 1,5-diphényl-1H-1,2,4-triazole-3-carboxamide de formule générale (I) suivante,

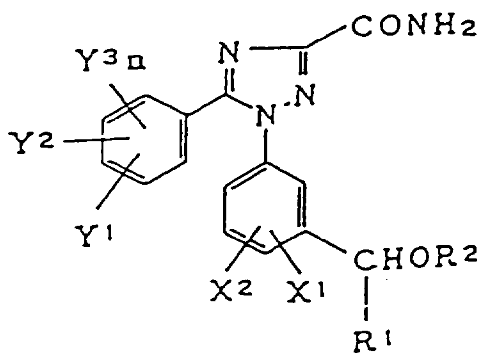

(I)

où $R^1$, $R^2$, $X^1$, $X^2$, $Y^1$, $Y^2$, $Y^3$ et n sont tels que défini dans la revendication 1, qui comprend :

la réaction d'un dérivé d'oxamide de formule (X) et d'un dérivé de benzaldéhyde de formule (XI) pour produire un dihydrotriazolecarboxamide de formule (XII) selon la formule de réaction suivante

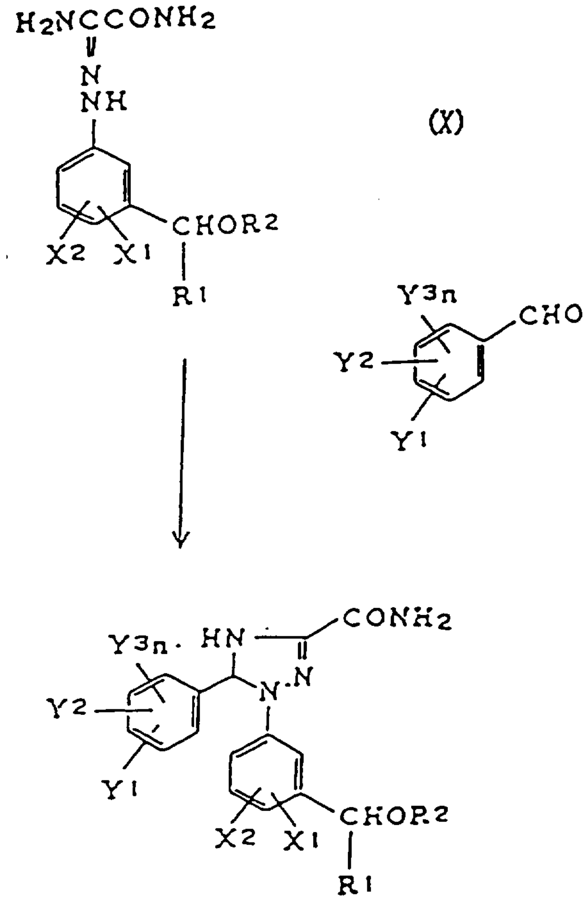

(X)

(XI)

(XII)

et

l'oxydation du dihydrotriazolecarboxamide.

**6.** Une composition antimicrobienne comprenant en tant que composé efficace au moins un dérivé de 1,5-diphényl-1H-1,2,4-triazole-3-carboxamide selon l'une quelconque des revendications 1 à 3.

**7.** Une composition antimicrobienne selon la revendication 6, qui est une composition antimicrobienne contre la moisissure grise.

**8.** Un procédé de préparation d'une composition antimicrobienne qui comprend le mélange d'au moins un composé selon l'une quelconque des revendications 1 à 3 à un support agricole ou horticole.

**9.** Un procédé pour le traitement antimicrobien d'une parcelle ou culture agricole ou horticole qui comprend l'application à la parcelle ou culture d'au moins un composé selon l'une quelconque des revendications 1 à 3.

**10.** Un procédé selon la revendication 9 pour la prévention ou le traitement de l'une ou plusieurs des maladies microbiennes suivantes : moisissure grise, rot gris, mildiou, rouille des feuilles et oïdium.